# EUROPEAN PATENT SPECIFICATION

(11) **EP 3 324 900 B1**
(45) Date of publication and mention of the grant of the patent: **14.08.2019**
(21) Application number: 16744489.2
(22) Date of filing: 22.07.2016
(51) Int. Cl.: A61F 5/37, A61M 5/52

(54) **ARM SUPPORT**
ARMSTÜTZE
SUPPORT POUR LE BRAS

(30) Priority: 22.07.2015 GB 201512934
(43) Date of publication of application: 30.05.2018
(73) Proprietor: Radial Cradle Ltd, Cambridge CB3 0DW (GB)
(72) Inventor: HOOLE, Stephen, Cambridge Cambridgeshire CB3 0DW (GB)
(74) Representative: Gill Jennings & Every LLP
(86) International application number: PCT/GB2016/052242
(87) International publication number: WO 2017/013444

(56) References cited:
- AT-B- 396 649
- US-A- 4 662 366
- US-A1- 2011 219 546

## Description

### Field

The invention relates to an arm support for supporting a patient's arm during a medical procedure. In particular, for supporting the arm in a position securely against the side of the patient's body whilst positioning the wrist so that a surgeon can access a radial site for performing a radial access procedure.

### Background

Investigating and treating coronary and structural heart conditions is possible using minimally invasive surgical techniques that access the heart retrograde through peripheral arteries. Traditionally, accessing the heart has been performed via the femoral artery in the leg due to its easy accessibility, and the fact that it is a large calibre vessel close to the heart. However, the femoral approach has some distinct disadvantages. The femoral artery is an end artery and traumatic dissection can lead to acute limb ischaemia, and there is an increase in life threatening bleeding complications e.g. retroperitoneal haematoma, particularly in emergency cases which can be difficult to manage. Furthermore, during the recovery period post procedure, the patient has to lie flat and recover in a bed, due to the location of the surgical access.

Recently, there has been a switch to using radial arterial access in Europe and some US centres to treat coronary and peripheral artery disease. The radial access provides distinct advantages over the well-established femoral artery route as the hand has a dual blood supply from both radial and ulnar arteries, which reduces acute ischaemic limb complications, with fewer bleeding complications owing to the easily compressible nature of the radial artery. Furthermore, in the radial access case, as the procedure is carried out on the patient's arm, rather than the leg as in the femoral access case, the patient can ambulate immediately after the procedure, and can be nursed during the recovery period in a chair rather than having to lie in a bed. These factors improve the patient experience and reduce health care costs as the length of hospital stay is shortened.

Approximately 350,000 coronary angiograms and 61,000 coronary angioplasty and stents are performed each year in the UK alone. Rates of radial access in the UK have increased over 10 years from 10 % in 2004 to 65 % in 2014 (BCIS data 2014). The increase in radial access usage is mirrored throughout Europe, in France and the Baltic countries radial access is the new preferred access site with greater than 90 % of cases performed radially. Adoption of the procedure in the US is now also growing.

Typically at present during the radial access procedure the arm is supported in a non-individualised ad-hoc manner, using a combination of pillows, foam and gel wrist supports and fixed arm boards that are co-registered to the table and bed which the patient lies on during the procedure.

As the arm boards are fixed at the height of the bed that they are attached to, and not the patient's body height, the arm position and therefore the operative field is not optimal, being below the body of the patient. This is known to lead to frequent back complaints amongst physicians. The low position of the arm during the procedure may also pose a risk of surgical implements and equipment used by the surgeon falling off the draped body, causing dislodging of the arterial access.

Furthermore, the radial access arm boards are not made to measure for each individual patient, which may mean that they are uncomfortable to use and as such the arm is poorly supported and may fall out of position, particularly if the patient is sedated. This can lead to destabilisation of interventional equipment inside the heart which may be hazardous to the patient. In addition, there currently does not exist an arm board that adequately supports the left arm, thus carrying out radial arterial procedures from the left arm carries increased risks. Further hazards may also arise from the fact that the arm boards are cleaned and reused between procedures, posing potential danger of infection.

Another disadvantage of such prior supporting devices is that the arm boards hold the arm at a position away from the patient's body. This prevents adequate shielding from both lead screens and the patient's body, resulting in increased radiation scatter and elevated radiation exposure to the treating physician/operator.

The poor support provided by the conventional equipment makes the radial arterial access procedure difficult and time consuming. Therefore, a need exists for providing improved support for the arm during the procedure that addresses all of the problems experienced by the current supporting means.

US 4 662 366 A is considered to represent the closest prior art, it discloses an immobilizing arm support for locating a patient's arm to facilitate angulated radiographic imaging. US 20150068534, US5601597, KR20140094961 also disclose example apparatus for use in surgery.

### Summary of Invention

According to a first aspect, there is provided an arm support for use in a medical procedure, comprising: an arm support strap and a hand support. The arm support strap comprises: a first end that is configured to pass around the arm of a patient and secure to a region of the arm support strap; a support region for supporting an arm of the patient, and a second end configured to pass across the torso of the patient to be secured so as to support the arm securely against a side of the torso in a position substantially co-planar with the torso. The hand support comprises: a first region adapted to secure the hand support to the arm support strap, and a second region adapted to engage with a hand of the patient for securing an associated forearm of the patient in a supinated, externally rotated position.

Due to the fact that the second end of the arm support strap is passed across the torso of the patient, the arm support registers the arm against the side of the patient's body in an elevated position. Advantageously, this provides an improved operating position for the surgeon than used in existing arm restraints.

The hand support ensures that the arm is secured in a supinated externally rotated position. The tension provided by the hand support between the first and second regions, causes the arm to be secured in a rotated position presenting the wrist and palm facing upwards. This enables easy access to the radial site during the radial procedure. Furthermore, this enables easy access for cleaning and draping over the exposed radial operative area.

The location of the arm supported against the body, with the second end of the arm support strap across the torso, helps provide a comfort feeling to the patient, cradling the arm. Moreover, since the arm support does not obscure the femoral area, ready access to the femoral artery is permitted.

Preferably, the support region is for supporting at least one of an elbow, upper arm, or the forearm of the patient.

Preferably, the second region of the hand support comprises a palm receiving portion. The palm receiving portion may comprise a palm strap configured to be wrapped around the palm and having means for securing the palm strap in position.

Preferably, the second region of the hand support comprises a thumb receiving portion. The thumb-receiving portion may comprise an opening in the second region of the hand support. Alternatively, or additionally, the thumb-receiving portion may comprise a thumb strap configured to pass underneath the hand and secure the thumb in position. Advantageously, the thumb receiving portion provides anchorage of the thumb, preventing the arm from rotating from its dorsiflexed position. Further advantageously, passing the thumb strap underneath the hand maintains a clear field on the palmer aspect of the wrist.

Preferably the first region of the hand support is removably attachable to the arm support strap. For instance, this attachment may be provided by adhesive strips on one, or both of the arm support and hand strap, such as hook and loop fasteners, for example Velcro™. Advantageously, in use, this enables continual adjustment of the hand strap ensuring the arm is maintained in the desired position. Alternately, any type of attachment could be envisaged for use. Alternatively, the hand support and arm support strap may be made from a unitary piece of material. For instance, in some embodiments the first region of the hand support may be unitary with the arm support strap.

Preferably, the support region comprises at least one perforation for registering the arm in position and providing flexibility to the arm support. Advantageously, this provides an intuitive region in which to place the arm when assembling the arm support before the radial procedure.

Preferably, the arm support is made of an absorbent, non-woven, low linting material that is strong in wet and dry conditions. The arm support may also be made of radiolucent material. Preferably, the arm supports are disposable. Advantageously, this may reduce the risk of infections.

Preferably, the arm support is reversible for use on both the left and the right arms. Advantageously, this allows the same arm support to be used on any arm.

Preferably, the second end of the arm support strap is configured to be secured to the patient's body, after passing across the torso.

Preferably, the second end of the arm support strap is configured to be secured to a bed or board under the patient, after passing across the torso. The second end of the arm support strap may be configured to attach back on itself. For instance, the arm support strap may be configured to pass through a slot on the board or bed before being attached back on itself. This provides a secure attachment.

Preferably, the second end of the arm support strap includes an adhesive patch for securing to the patient, or to a bed or board under the patient. Alternatively, or additionally, the second end of the arm support strap comprises at least one hole for securing to an anchor post.

Preferably, the arm support according to the first aspect of the invention, further comprises a secondary retention strap, the secondary retention strap comprising: a first end that is configured to be secured to a bed or board under the patient; and a second end that is configured to attach to the arm support; such that the secondary retention strap prevents the arm from moving. Advantageously, the secondary retention strap further secures the arm support strap in position.

Preferably, the second end of the arm support strap can be configured to be attached around the neck of the patient to form a sling. In some example arm supports the arm support strap may comprise a perforation for receiving the head of the patient when forming a sling.

Preferably, the first end of the arm support strap is configured to pass underneath and then back over the arm of the patient before being secured. Advantageously, this causes the arm support strap to form a cradle like region surrounding and supporting the arm.

Preferably, the region of the arm support strap to which the first end is configured to secure, is a region of the arm support strap that passes across the torso of the patient. Advantageously, this location of attachment promotes the arm being in a high elevated position with respect to the body of the patient.

Preferably, the first end of the arm support strap comprises at least one adhesive patch for securing to the region of the arm support strap. The at least one adhesive patch for securing to the region of the arm support strap may be an elongate strip. The region of the arm support strap may comprise at least one adhesive patch for securing to the first end of the arm support strap. The at least one adhesive patch for securing to the first end of the arm support strap may be an elongate strip. Alternatively, the first end of the arm support strap and said region of the arm support strap each comprise at least one elongate adhesive patch for attaching to one another, wherein the at least one elongate adhesive patch of the arm support strap and said region of the arm support strap are orientated at an angle to one another, whereby the arm support is adjustable to accommodate a range of patient sizes.

Preferably, the second end of the arm support strap comprises at least one upper anchor strap, and the first end of the arm support strap comprises an associated upper anchor slot, wherein the arm support further comprises at least one external rotator strap, and wherein the support region is an elbow support region.

Preferably, when the second region of the hand support comprises a thumb receiving portion, and the hand support and arm support strap are made from a unitary piece of material, the at least one upper anchor strap has a free end that is configured to pass through the associated slot and across the torso of a patient to be secured so as to support the arm securely against a side of the torso in a position substantially co-planar with the torso; wherein the at least one external rotator strap has a free end that is configured to be secured so as to fix the forearm in an externally rotated position.

Preferably, the thumb-receiving portion is located on the opposite side of the arm support to the at least one upper anchor strap.

Preferably, the second end of the arm support comprises the at least one external rotator strap. The external rotator strap helps further maintain the arm in the external rotated dorsiflexed position. The external rotator strap may have an associated external rotator slot. Alternatively, or additionally, the second end of the arm support strap comprises a fore external rotator strap, and a rear external rotator strap.

Preferably, the thumb-receiving portion is a thumb support strap which branches off from the fore external rotator strap. Alternatively, the thumb receiving portion may be a palm receiving portion.

Preferably, the at least one upper anchor strap is in between the fore external rotator strap and rear external rotator straps.

Preferably, the free end of the at least one external rotator strap is configured to be secured to the body of the patient after passing across the torso of the patient. Alternatively, or additionally, the free end of the at least one external rotator strap is configured to be secured after passing across the torso of the patient, to a bed the patient is on. The securing to the patient and/or bed may be provided by an adhesive patch. Alternatively, or additionally, the at least one external rotator strap comprises at least one hole for securing the free end thereof to an anchor post. Alternatively, or additionally, the at least one external rotator strap comprises an adhesive patch for securing the free end thereof to the at least one external rotator strap after passing through a slot.

According to a second aspect there is provided a package comprising a plurality of arm supports according to the first aspect. Preferably, the plurality of arm supports are provided on a roll, individual arm supports configured to be separable from one another along a line of weakness. Advantageously, supplying the arm supports on a roll allows easy dispensing of the arm supports in the surgical environment, and aids in their transportability.

Preferably, when the first region of the hand support is removably attachable to the arm support strap, the arm support strap and the hand support are separable from one another along a line of weakness when provided on a roll.

According to a third aspect there is provided a kit of parts for use in a medical procedure, comprising: the package according to the second aspect and a securing device, wherein the securing device is configured for securing the second end of the arm support strap.

Preferably, the securing device is for secure attachment to a bed under the patient. Alternatively, the securing device is a secure attachment to a board under the patient.

Preferably, the securing device comprises at least one anchor rod, for receiving at least one hole on the second end of the arm support strap to provide secure attachment.

Preferably, the securing device comprises at least one spike configured to pierce the second end of the arm support strap to provide secure attachment.

Preferably, the securing device comprises a clamp configured to grip the second end of the arm support strap to provide secure attachment. The clamp may comprise gripper teeth.

Preferably, the securing device comprises a slot configured to receive the second end of the arm support strap, before being attached back on itself. The slot may be a slot on a board, the board for placement under the patient's body during the radial procedure.

According to a fourth aspect not falling under the scope of the present invention, there is provided a method of using the arm support of the first aspect, the method comprising; placing the arm of a patient on the support region of the arm support strap, passing the first end of the arm support strap around the arm of the patient and securing to a region of the arm support strap, passing the second end of the arm support strap across the torso of the patient; securing the second end so as to support the arm securely against a side of the torso in a position substantially co-planar with the torso, engaging the hand of the patient with the second region of the hand support, securing the first region of the hand support to the arm support strap, such that the associated forearm of the patient in a supinated, externally rotated position.

Preferably, the securing device may also be for securing the external rotator strap.

Embodiments of the present invention provide an arm support for use in medical procedures. The arm support is particularly suitable for investigating and treating coronary and structural heart conditions using radial arterial access procedures, for securely supporting the arm securely against the side of the torso and rotated to a position that facilitates ease of access to the radial artery site, for example, during the procedure, and overcomes at least some of the problems experienced by known supporting techniques for this purpose.

The scope of the present invention is defined by the appended claims.

### Description of Drawings

The invention will be described, by way of example, with reference to the accompanying drawings, in which:
Figures 1A and 1B show front, and back views respectively, of an arm support according to a first embodiment of the present invention;
Figure 2 shows a top down view of a board for use with the arm support according to the first embodiment of the present invention;
Figure 3 shows the arm support of Figure 1 arranged for use in combination with the board of Figure 2;
Figures 4A and 4B show front, and back views respectively, of an arm support according to a modified version of the first embodiment of the present invention;
Figures 5A and 5B show two views of the arm support of Figures 4A and 4B arranged for use in combination with the board of Figure 2;
Figure 6 shows the arm support of Figures 4A and 4B when in use for carrying out a radial procedure on a patient;
Figure 7 shows a transverse cross section of the arm support according to the first embodiment of the invention, when in use;
Figure 8 shows a view from above of the arm support according to a first embodiment of the invention, when in use;
Figure 9 shows a view from above of the arm support according to a variant of the first embodiment of the invention, when in use;
Figures 10A and 10B show a close up view of the hand strap in Figure 8, with and without the hand, respectively;
Figure 10C shows various alternative thumb holes for use with the hand strap shown in Figure 8;
Figures 11A and 11B show a close up view of the hand strap in Figure 9, with and without the hand, respectively;
Figure 12 shows a variant of the arm support strap of Figure 1 or Figure 4 that is suitable for use as a sling for the patient's arm after the radial procedure;
Figures 13A and 13B show front, and reverse views, respectively, of the patient with the arm support strap of Figure 12 in use as a sling post radial procedure;
Figure 14 shows a side elevation of the sling adaptation shown in Figure 13A and 13B;
Figures 15A and 15B show an alternative sling adaptation of the arm support, showing front and back views of the patient, respectively;
Figure 16A shows a package comprising a plurality of arm supports according to all of the embodiments of the invention, on a roll;
Figure 16B shows a package comprising a plurality of arm supports according to the first embodiment of the invention, on a roll;
Figure 17 shows an arm support according to a second embodiment of the invention;
Figure 18 is a view from above of the arm support according to a second embodiment of the invention, in use;
Figure 19 is a side view of the arm support according to the second embodiment of the invention, in use;
Figure 20 shows a transverse cross section of the arm support according to the second embodiment of the invention, in use;
Figures 21A-D show different possible attachment means for the arm support according to the invention;
Figure 22 shows an arm support according to a third embodiment of the invention;
Figure 23 is a view from above of the arm support according to a third embodiment of the invention, when in use; and
Figure 24 shows a package comprising a plurality of arm supports according to either the second or third embodiment of the invention, on a roll.

### Detailed Description

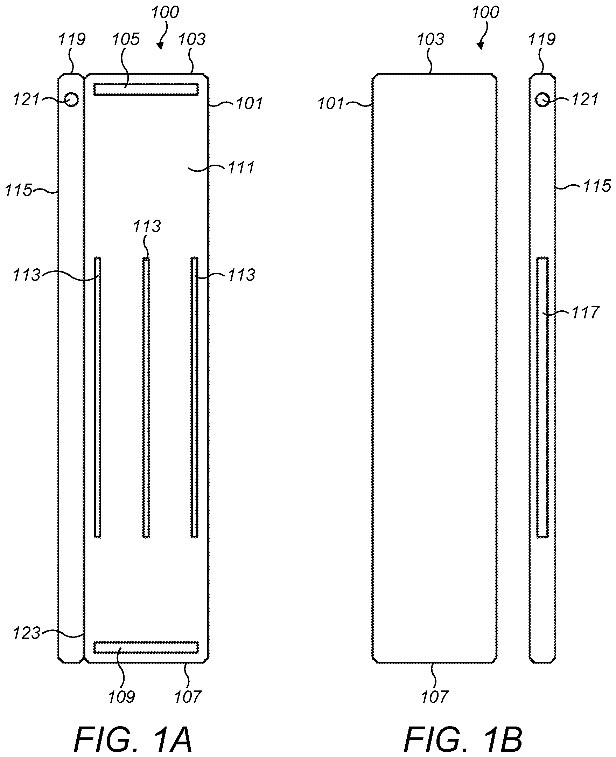

Figures 1A and 1B illustrate front, and back views respectively, of an arm support 100 according to a first embodiment of the present invention, showing a 'blank' of the arm support 100 in flat condition when not in use.

The arm support 100 comprises an arm support strap 101. The arm support strap has a first end 103 and a second end 107, distal to one another. As can be seen from Figure 1A the front of the arm support strap 101 comprises an elongate adhesive strip 105, along the width of a first end of the arm support strap 103. A second elongate adhesive strip 109 is positioned along the width of a second end of the arm support strap 107.

Located on the front of the arm support strap 101, between the first 103 and second 107 ends, are a number of adhesive strips 113. In the embodiment shown in Figure 1A, three elongate strips of adhesive material are present; however any number of adhesive strips could be used. The adhesive strips 113 extend along the length of the arm support strap 101, and thus are orientated perpendicular to adhesive strips 105, 109. The adhesive strips shown in Figure 1A and 1B are of the hook and loop type, for instance Velcro™.

An arm support region 111 is located between the first end 103 of the arm support strap and the strips of adhesive material 113. The arm support 100 also includes a hand support strap 115. Figure 1A illustrates the arm support 100 before use, where the hand support strap 115 is attached to the arm support strap 101 along perforation 123. In Figure 1B, the hand support strap 115 has been removed from arm support strap 101 along perforation 123, so that it is ready for use.

As can be seen in Figure 1B the back of the hand support strap 115 contains an adhesive strip 117. The adhesive strip 117 extends along a region of the length of the hand strap 115. A thumb hole 121 is located at a thumb receiving end 119 of the hand support strap 115. The thumb hole 121 extends through the hand strap 115, as can be seen in Figures 1A and 1B.

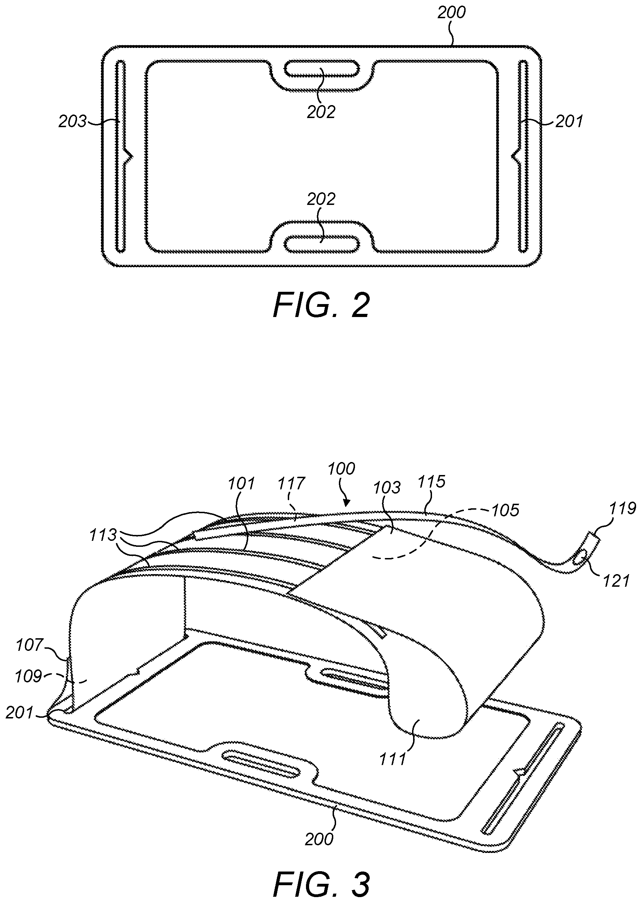

Figure 2 illustrates a rectangular board 200 for use with the arm support 100. Slots 203 and 201 that extend through the board are located along each shortest side of the board 200. A handle 202 is located on each of the longest sides of the board, for carrying the board when not in use. Alternatively, no handles, or only one handle may be required. Preferably, the board is made from a machined polymer sheet; however any suitable material could be used. Although not shown in Figure 2, the board may have elbow alignment indicators, to help with easy alignment of the patient's arm.

Figure 3 shows the arm support 100 of Figure 1 arranged for use in combination with the board 200 of Figure 2. As shown, the hand support 115 has been removed from the arm support strap 101, along the perforation 123 shown in Figure 1B. The adhesive strip 117 is connected to at least one of the adhesive strips 113, such that the hand strap 115 is attached to the arm support strap 101. The thumb receiving end 119 of the hand support strap 115 is not attached to anything and is therefore free.

The second end 107 of the arm support strap 101 is attached back onto the arm support strap 101 after being passed through slot 201 in the board 200. The attachment is provided by the adhesive strip 109 and the adhesive strips 113 engaging one another.

The first end 103 of the arm support strap 101 is folded such that the adhesive strip 105 and the adhesive strips 113 engage one another. This forms a loop region of the arm support strap, which encompasses the arm support region 111. The loop region is of a size for accommodating the arm of a patient.

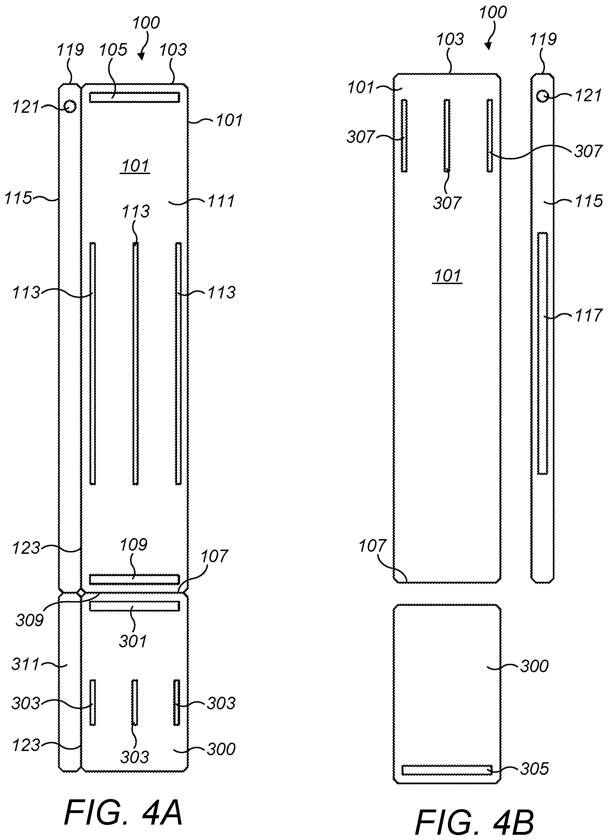

Figure 4A shows a front view of an arm support 100 according to a modified version of the first embodiment of the present invention, showing a 'blank' of the arm support 100 in flat condition when not in use. The hand support strap 115 is connected to the arm support strap 101 along perforation 123. A further secondary support strap 300 is also attached to arm support strap 101 along perforation 309.

Figure 4B shows a rear view of the arm support 100 of Figure 4A, when ready for use. The hand support strap 115 has been removed from the arm support strap 101 along perforation 123, and the secondary support strap 300 has been removed from arm support strap 101 along perforation 309. Portion 311 has been removed along perforations 123 and 309 and has been discarded.

The features of the arm support 100 shown in Figures 4A and 4B that are common with the arm support 100 shown in Figures 1A and 1B will not be discussed further here.

As shown in Figure 4A, the front of the secondary support strap 300 comprises an elongate adhesive strip 301, along the width of one end of the secondary support strap 300. In addition, a number of adhesive strips 303 are located on the front of the secondary support strap 300. In the embodiment shown in Figure 4A, three adhesive strips 303 are present; however any number of adhesive strips 303 could be used. The adhesive strips 303 extend along the length of the secondary support strap 300, thus they are orientated perpendicular to the adhesive strip 301.

On the reverse of the secondary support strap 300, is an elongate adhesive strip 305, located along the width of the opposite end of the secondary support strap 300 to adhesive strip 301.

As can be seen in Figure 4B, on the reverse of the arm support strap 101 is a number of elongate adhesive strips 307. In the embodiment shown in Figure 4B three adhesive strips 307 are present, however any number of adhesive strips 307 could be used. The location of the adhesive strips 307, on the reverse of the arm support strap 101, corresponds to the arm support region 111 located on the opposite side of the arm support strap 101.

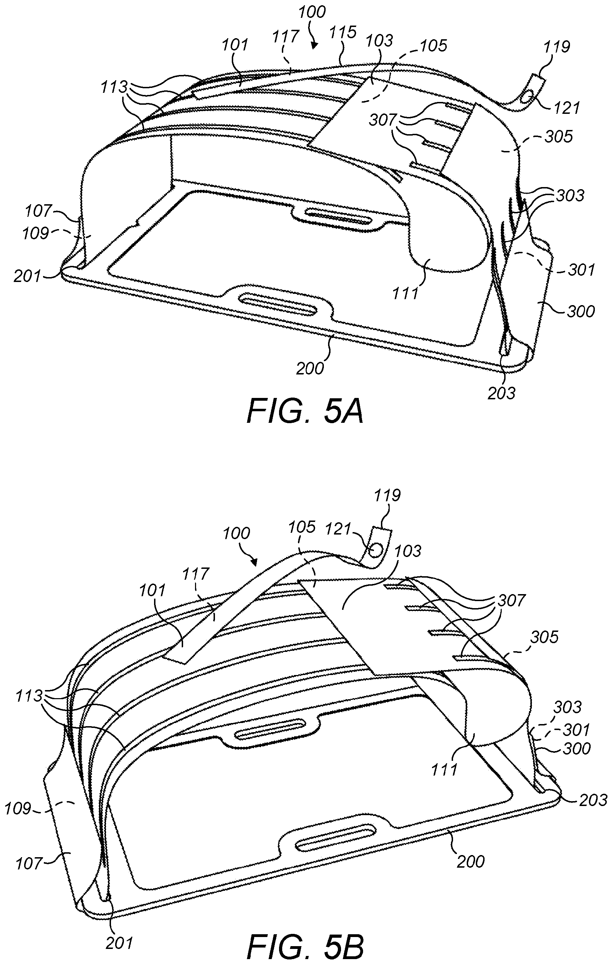

Figures 5A and 5B show two views of the arm support 100 shown of Figures 4A and 4B, arranged for use in combination with the board 200 of Figure 2. A description of the features of Figures 5A and 5B common with Figure 3 will not be repeated here.

One end of the secondary support strap 300 is attached back onto itself after being passed through slot 203. The attachment is provided by the adhesive strip 301 and the adhesive strips 303 engaging one another. The other end of the secondary support strap 300 is attached to the arm support strap 101. This attachment is provided by the adhesive strip 305 and the adhesive strips 307 engaging one another.

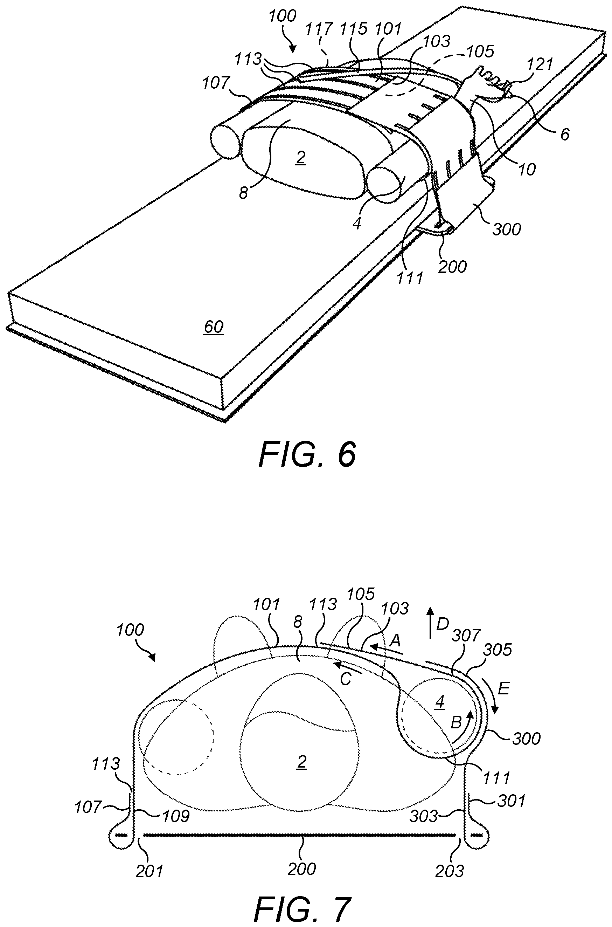

Figure 6 illustrates the arm support 100 of Figures 4A and 4B, when in use for carrying out a radial procedure, on a patient 2. As can be seen, the board 200 is placed under the bed 60 on which the patient 2 is lying.

The arm support 100 is assembled as described previously for Figure 5A and 5B. This will now be described in relation to the body of the patient 2. The second end 107 of the arm support strap 101 has been passed across the torso 8 of the patient, and attached to itself, as described previously for Figures 5A and 5B. The arm 4 of the patient, on which the radial procedure is to be performed, is placed on the arm support region 111. The first end 103 of the arm support strap 101 has been passed back over the top of the arm 4 of the patient and secured to the arm support strap 101 using adhesive strips 113 and adhesive strip 105. This provides a tight fit around the arm 4 of the patient to keep the arm secure. The tension of the arm support strap, and the positioning of the arm 4, can be adjusted by repositioning the location along attachments 113 to which attachment 105 is attached.

The thumb 6 of the patient is within the thumb hole 121, anchoring the thumb 6 of the patient. The hand strap has been passed under the hand of the patient. The hand strap 115 is attached to an exposed region of the adhesive strips 113 using adhesive strip 117. The attachment of adhesive strip 117 and adhesive strips 113 can be adjusted to ensure the hand of the patient is brought under tension in a dorsiflexed position, providing access to the radial site 10. The arm support strap 101 is secured using the secondary support strap 300 as previously described for Figures 5A and 5B.

Final adjustments can be made to the positioning of the attachments of; the arm support strap 101 to itself cradling the arm 4, the hand support strap 115 to the arm support strap 101, and the secondary support strap 300 to the arm support strap 101. This ensures a secure attachment of the arm 4 against the side of the torso 8, securing the forearm in a supinated externally rotated position. This provides easy access for the surgeon to the radial site 10.

The location of the arm 4 is typically within one arm thickness of the top of the patient's torso 8 when the patient is lying down, secured anywhere between either one arm 4 thickness above the top of the torso 8 or one arm 4 thickness below the top of the torso 108 in this orientation. The relatively high position of the arm 4 in relation to the body of the patient 2 advantageously provides the physician with an improved operating position.

Figure 7 illustrates a cross section free body diagram of the arm support 100 in use, on the body of a patient 2. The forces provided by the arm support 100 due to its specific orientation and securement are also shown. Forces A and B indicate the tension provided by the first end 103 of the arm support strap 101, that extends under and then back over the arm 4 of the patient, before being secured to itself by adhesive strips 113 and 105. Force C is the tension provided by the second end 107 of the arm support strap 101 that has been extended across the torso 8 of the patient, before being attached back on itself through slot 201.

The tension provided by the forces through the straps can be resolved into horizontal and vertical components. The tension provided by Forces A, B and C have a horizontal component that urges the arm 4 towards the patient's side, as well as a vertical component that urges the arm 4 upwards, against the weight of the arm 4 which is in the opposing vertical direction. Force D represents the combined effect of the vertical components of the tension provided by the first 103 and second end 107 of the arm support strap 101, resulting in the upwards force that maintains the arm 4 in the preferred high orientation relative to the torso 8 of the patient. Force E represents the tension provided by the second support strap 300, maintaining the arm 4 of the patient in a position at the side of the patient. This prevents the patient from getting up. The vertical component of Force E balances with Force D maintaining the arm 4 in position.

Both the secondary support strap 300 and arm support strap 101 are attached to the board 200 as described for Figures 5 and 6. The weight of the patient's torso 8 is supported by the bed on which the patient is lying (not shown in Figure 7). The arrangement of the arm support strap 101 across the torso 8 of the patient helps to distribute the load of the patient's arm 4 to the bed, which is maintained in position by the securing to the board 200.

Although Figures 6 and 7 have been described in relation to the variant of the first embodiment shown in Figures 4A and 4B, it can be readily understood that the description can also apply to the variant of the first embodiment shown in Figures 1A and 1B. The difference between the two variants is the inclusion of a secondary support strap 300, for providing additional support to secure the arm support strap 101 in place.

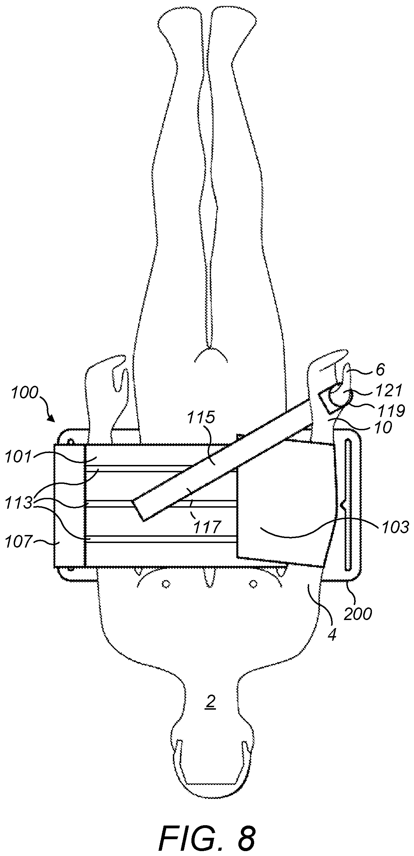

Figure 8 shows a top down view of the arm support 100 of Figure 1, when in use on patient 2. The arm support 100 is assembled as described in Figures 5 and 6, without the secondary support strap 300 present. The hand support strap 115 can be clearly seen with the thumb 6 of the patient received within the thumb hole 121. As described, the tension of the hand support strap 115 on the hand causes the arm 4 to be rotated in a supinated orientation, i.e. palm upwards. The radial site 10 is readily accessible with this arrangement as can be seen.

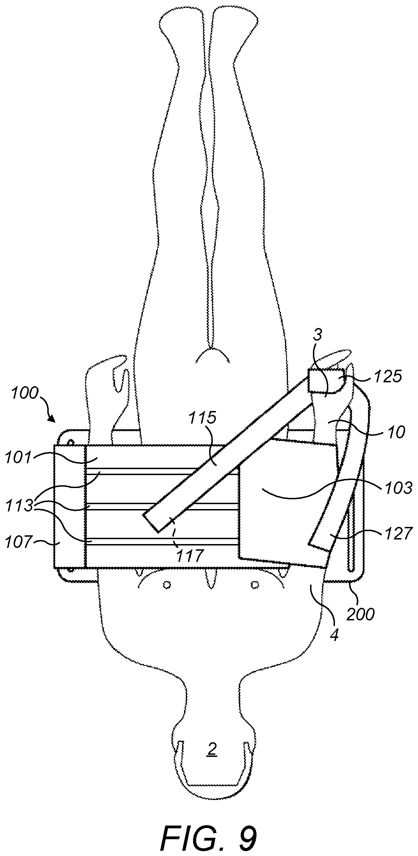

Figure 9 shows the same top down view as shown in Figure 8, of a further variant of the arm support 100 as shown in Figure 1. The arm support 100 shown in Figure 9 has a different configuration of hand strap 115, than shown in Figures 1 to 8. As with the variants shown in Figures 1 to 8, the hand strap 115 in Figure 9 attaches to the arm support strap 101 by attachment 117 and 113. However, rather than a thumb hole 101 for receiving the thumb 6 of the patient, the hand strap 115 in Figure 9 has a palm receiving portion 125 for receiving the palm 3 of the patient. The arrangement of the palm receiving portion 125 will be described in more detail below. A second end 127 of the hand support strap 115, distal to the end of the hand support strap 115 having the adhesive strip 117, is attached to the rear of the arm support strap 101, in a location above arm 4 of the patient. Although no attachment mechanism is explicitly shown in Figure 9 between the second end 127 and the arm support strap 101, it can be envisaged that a similar attachment mechanism to adhesive patches 117 can be used. For instance, the second end 127 of the hand strap 115 may in some variants have an adhesive strip for attaching to the attachment strips 307, so as to attach the second end 127 of the hand strap 115 to the back of the arm support strap 101 as shown in Figure 4 and 5.

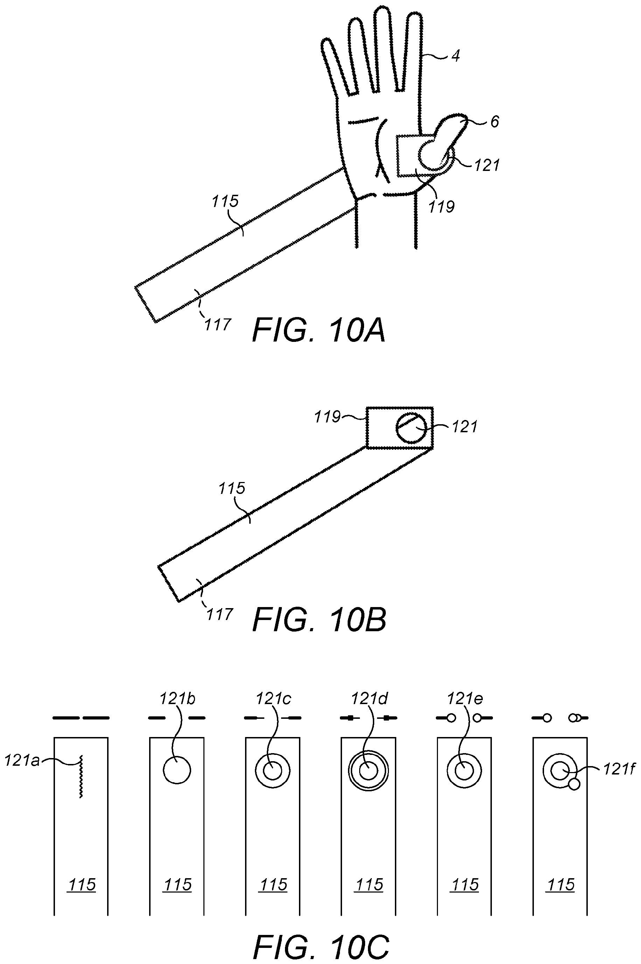

A more detailed view of the hand strap 115 of Figure 8 is shown in Figure 10A and 10B, with and without the hand 4, respectively. The thumb 6 of the patient is received in the thumb hole 121, located on the thumb receiving end 119 of the hand support strap 115. Subsequently, the hand support strap 115 passes underneath the hand, before attaching to the arm support 101 as previously described. Advantageously, having the hand support strap 115 passing underneath the hand maintains a clear field on the palmer aspect of the wrist, as required for the radial procedure.

Various alternative thumb holes 121a-f are illustrated in Figure 10C. Cross sections of the various thumb holes are also shown. A thumb hole formed from a perforated slot is shown as 121a. A thumb hole formed from a perforated disc is shown as 121b. A thumb hole formed from a silicon insert is shown as 121c. A thumb hole formed from a silicon insert with co-moulded ridged rim is shown as 121d; the rigid rim ensures shape retention. A thumb hole formed from a solid foam ring is shown as 121e. A thumb hole formed from a user inflatable PVC ring is shown as 121f; this allows the size of the thumb hole to be varied to accommodate a range of thumb sizes. The various thumb holes described here may reinforce the thumb hole, and may provide padding to comfortably support the thumb. Any type of hole for accommodating the thumb could be envisaged, such that it secures the hand in the dorsiflexed orientation.

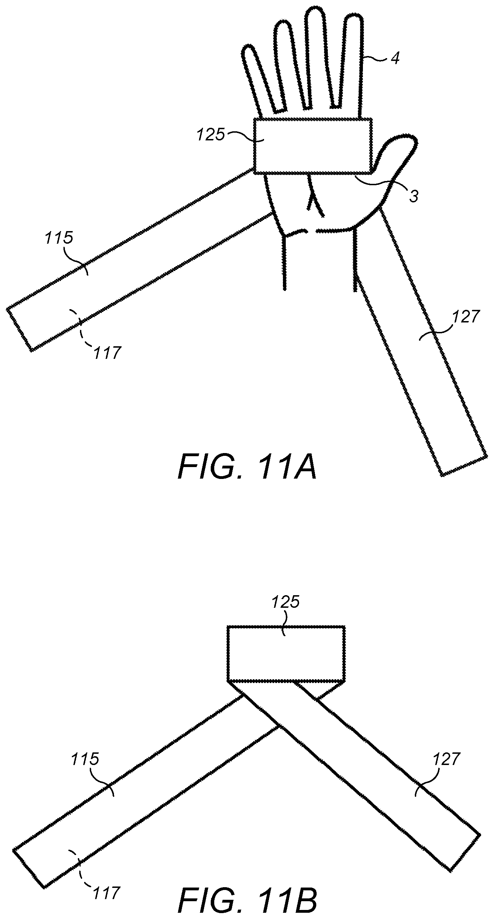

A more detailed view of the hand strap 115 in Figure 9, is shown in Figure 11A and 11B, with, and without the hand 4, respectively. The hand strap 115 takes the form of a palm strap, which is looped around the hand of the patient, specifically across the palm. Both ends of the hand support strap 115, i.e. the end containing the adhesive strip 117, and the second end 127, are attached to regions of the arm support strap 101, as described above in relation to Figure 9. The palm receiving portion 125 exerts a tension on the hand of the patient, which can be adjusted by the repositioning of the attachments between the hand support strap 115 and the arm support strap 101. This tension achieves the same result as the thumb type strap shown in Figure 10, by positioning the hand in a dorsiflexed configuration for easy access to the radial site. Both ends of the hand support strap 115 pass underneath the hand of the patient before being secured to the arm support strap 101. This prevents the hand support strap 115 from obstructing the radial site.

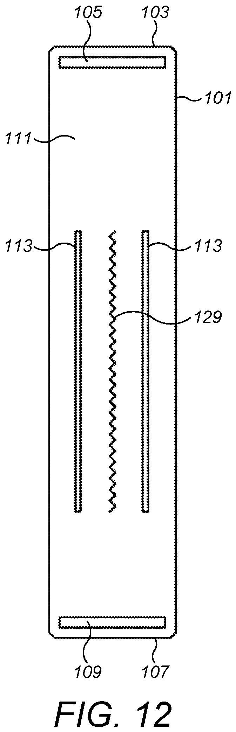

Figure 12 illustrates a variant of the arm support strap 101 of Figure 1, or Figure 4, that is suitable for being used as a sling for the patient's arm after the radial procedure. Rather than having three adhesive strips 113 running down the length of the arm support strap 101, as in Figure 1 and 4, the middle adhesive strip has been replaced by a perforation 129. In alternative hand supports 100, the inclusion of a perforation 129 may be in addition to three or any number of adhesive strips 113.

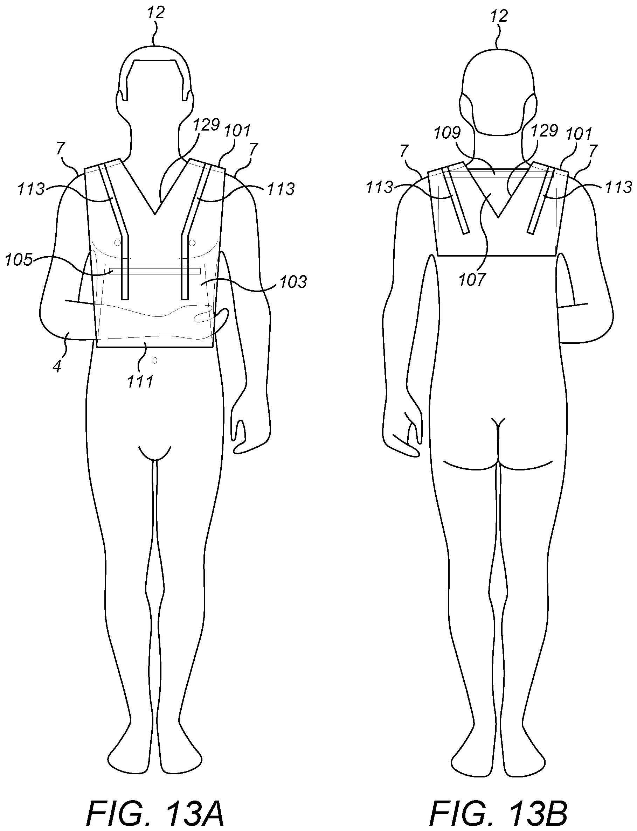

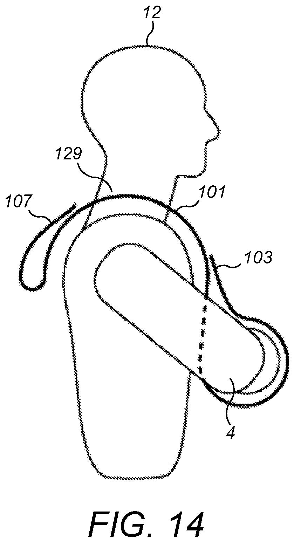

Figures 13A and 13B show front and reverse views, respectively, of the patient with the arm support strap 101 of Figure 12 being used as a sling post radial procedure. After the procedure has been completed, the patient will be located in the position as shown in Figure 6 and 7. The second end 107 of the arm support strap is then detached from itself, and passed back through slot 201. The hand support strap 115 is then disconnected from the arm support strap 101, and from the patient's hand. The first end 103 of the arm support strap 101 remains connected to itself, such that the arm support region 111 cradles the arm 4 of the patient. If the arm support 100 contains the secondary support strap 300, this is also detached. The central perforation 129 of the arm support strap is then split to create a head slot. The head 12 of the patient is then passed through the perforated head slot 129, the arm support strap 101 rests on the shoulders 7 of the patient. The second end 107 of the arm support strap 101 is pulled down the back of the patient to adjust the height of the patient's arm 4. The second end 107 of the arm support strap 101 is then folded back on itself and attached via adhesive strips 109 and 113; this prevents the arm support strap 101 from moving forward, due to the second end 107 of the arm support strap 101 resting against the back of the neck of the patient. Figure 14 illustrates a side elevation of the sling adaptation of the arm support 100 shown in Figure 13A and 13B.

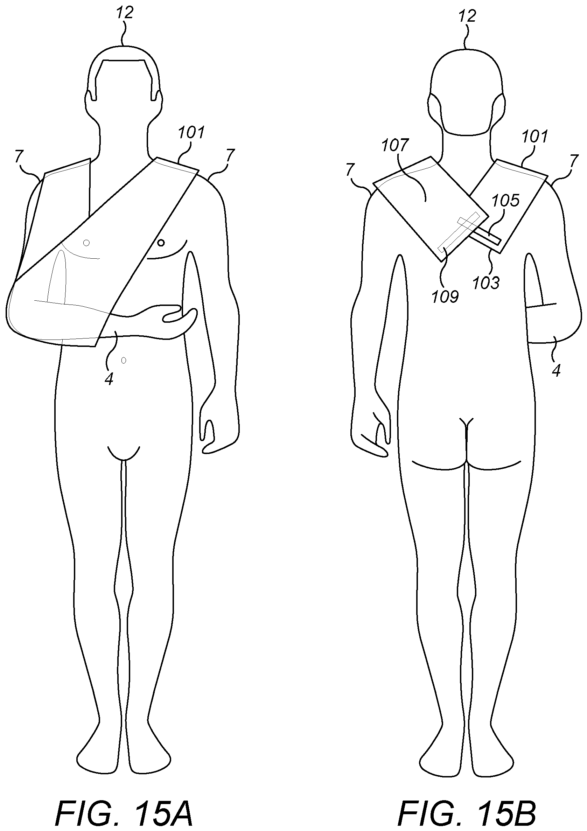

An alternative sling adaptation of the arm support 100 is shown in Figure 15A and 15B, showing front and back views of the patient, respectively. Rather than requiring a perforation 129, as in Figures 12 to 14, the arm support strap 101 shown in Figure 1 and Figure 4 can be used to form the sling shown in Figure 15 with no further adaptation to the arm support strap 101 needed. After the radial procedure has been completed, as mentioned above, the patient will be located in the position as shown in Figures 6 and 7. The second end 107 of the arm support strap is then detached from itself, and passed back through slot 201. The hand support strap 115 is also disconnected from the arm support strap 101 and removed from the patient's hand. If the arm support 100 contains the secondary support strap 300, this is also detached. The first end 103 of the arm support strap 101 is then detached from itself, and passed back through slot 203. The arm support strap 101 is laid out flat, as shown in Figures 1 and 4. The elbow of the patient, on which the procedure has been carried out, is placed in the centre of the arm support strap 101. Both the first 103 and second ends 107 of the arm support strap 101 are passed over alternate shoulders 7 of the patient. The two ends 103, 107 of the arm support strap 101 are attached to one another behind the patient using adhesive strips 105 and 109. A sling is formed holding the arm in position across the front of the patient.

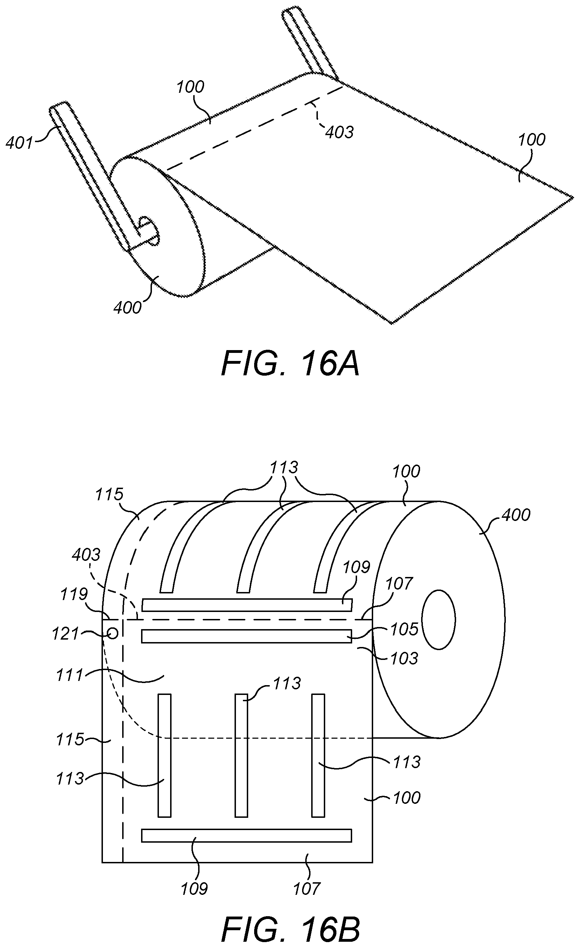

A number of arm supports 100, according to the embodiments described, can be provided on a roll 400 as illustrated in Figure 16A. The roll 400 comprises a series of arm support blanks 100, attached together with perforations 403, or some other line of weakness between adjacent arm supports 100 to provide easy detachment from the roll 400 when required. Upon pulling an arm support 100 from the roll 400, the perforations 403 provide a line of weakness in the material allowing the arm support 100 to be detached from the roll 400.

Preferably, the arm supports 100 are formed from the same piece of material. Advantageously, this means that the arm support 100 can be manufactured and provided cheaply, and in bulk, requiring little assembly before use. Preferably, the arm supports are disposable after each use. Advantageously, this may reduce the risk of infections. In one embodiment the roll 400 can hold fifty arm supports, however the roll may hold various numbers of arm supports. Furthermore, board 200 could be placed within the middle of the roll 400 when not in use. Advantageously, this provides easy transport of the arm supports 100 and board 200.

The roll 400 can be placed on a dispenser 401 for convenient dispensing in the surgical environment.

Figure 16B shows a roll 400 containing the arm support 100 of Figure 1. The features of the arm support 100, such as the thumb hole 121, may already be cut out on the arm support blanks 100. However, they may alternatively be provided as perforations to be pushed out after dispensing of the arm support 100 from the roll 400. The adhesive strips 105, 109, 113, 117, 301, 303, 305, 307 may be included on the arm support strap 100 when on the roll 400. To avoid the problem of the rolls 400 being too bulky, due to the additional thickness from the adhesive strips, the arm supports 100 may contain recesses for receiving the adhesive strips of adjacently rolled arm supports 100, to minimise thickness. Alternatively, the adhesive strips may come separately and have to be placed onto the arm support 100 before use. A similar roll 400 to that shown in Figure 16B would be used for the variation of the arm support 100 including the secondary support strap 300 as shown in Figure 4, or the arm support 100 which the palm strap 115, as shown in Figure 9.

Preferably the adhesive strips 105, 109, 113, 117, 301, 303, 305, 307 used on the arm support 100 in Figures 1 to 16 are hook and eye fasteners, such as Velcro™. Advantageously, this allows continual detachment and re-attachment of the arm support. This enables the position of the arm support 100 to be adjusted such that the optimal position of the arm of the patient is maintained, whilst providing comfort to the patient. This allows fine tuning the balance of orientation and tension of the arm support 100. Alternatively, any other type of attachment known in the art could be used, for instance sticky tape, Omni-tape® and heavy duty adhesive sticker areas. Advantageously, the attachments are repositionable to achieve the most secure and comfortable fit of the arm support 100.

During the radial access procedure, a catheter is inserted into the radial artery in the patient's arm 4. Advantageously, the arm support 100 according to the described embodiments provides a clear distal zone for inserting the catheter into the arm 4, with the arm support strap 101 and the hand support strap 115 orientating the arm 4 such that the radial artery is easily accessible throughout the procedure. Although the arm support strap 101 is shown in the Figures as a rectangular blank, in alternative example arm supports 100 the arm support strap 101 may be of various shapes. In arm supports where the arm support strap is large enough to extend to the arm of the patient, the region of the arm support strap nearest to where the hand of the patient is received may be templated to the hand, and may be a cutaway. Advantageously, this helps further provide a clear access to the radial operative area, for instance enabling cleaning and draping.

Preferably, the arm support 100 is made from hypoallergenic, low weight, flexible, absorbent material. The material preferably has an absorbent upper surface and a waterproof under surface that resists fluid-strike through the material, and prevents body fluid run off during the procedure. Preferably the arm support 100 is disposable, reducing the risk of infections. Preferably the arm support 100 is made from a non-woven, low linting material that is strong in wet and dry conditions. Preferably it will meet flammability class 1. Preferably it will be a material such as Steri-Drape™.

During the radial access procedure monitoring of the catheter is provided by x-rays or other radiation detection methods, therefore the arm support 100 is preferably made from a radiolucent material to enable full visualisation of the heart through the arm support 100 by fluoroscopy.

It is intended that the arm support 100 is reversible for use on both the left and right arms. As can be identified from Figures 1 and 4 the arm support strap 101 can be orientated such that it can be used on either arm of the patient.

The arm support 100 according to embodiments provides passive support to the patient even when unconscious, providing support from the underside of the arm support 100, with the load from the arm distributed by the arm support strap 101 when under tension. Advantageously, rather than the arm being attached directly to the bed frame on the same side of the patient's body as the arm 4 on which the procedure is being carried out (as is common in current radial access techniques), the arm support 100 provides attachment over the body of the patient ensuring the arm is always maintained in the same plane as the patient's body. This additionally causes the arm to be raised and supported in a high position against the patient's body, rather than located in a low orientation attached directly to the bed. This provides the advantage of the arm being at a more desirable operating position for the physician, lowering their risk of developing back complaints from carrying out multiple procedures over time. Furthermore, due to the high arm position and the arm support strap, surgical drapes across the arm and torso are approximately flat, or may even have a dip in them, in sharp contrast to the existing situation in which drapes have a tendency to form a tent-like surface from which any surgical tools or equipment that may be rested on the drapes are prone to sliding off.

Advantageously, passing the second end 107 of the arm support strap over the body of the patient, before attaching to the board 200, helps to prevent the patient from sitting up during the procedure. This is further prevented by using the secondary support strap 300.

Advantageously, the arm support 100 can be used on patients of any size with the arm support 100 adaptable to specific patients, unlike the systems used in current radial access techniques. This is achieved by the arm support strap 101 and hand support strap 115 having a length sufficient to secure any patient size with attachment means ensuring the desired tension can always be achieved. Furthermore, boards and straps may be manufactured in various sizes, to accommodate various patient sizes.

Advantageously, the arm support 100 permits easy access for defibrillator pads and ECG lead electrode attachments to the chest, for monitoring and treatment of electrical disturbance during the interventional procedure. The defibrillator pads may be applied before securing the arm support 100 to the patient. Advantageously, performing defibrillation whilst the arm is located in the arm support 100 prevents the arm from flailing around during shocks which could damage equipment. Moreover, the adhesive strips may permit rapid removal of the arm support if necessary, or alternatively the arm support 100 may be easily cut if quick removal is required.

Many modifications and variations may be made to the above-described embodiment within the scope of the invention.

The arm support region 111 as shown in Figures 1 and 4 is a region of the arm support strap 101 for receiving the arm of the patient. Preferably, the elbow of the patient is received on the arm support region 111, however; any part of the arm may be received, including the forearm or the upper arm in addition to, or instead of the elbow of the patient.

A cushioning pad may be located on the arm support region 111, for registering the arm of the patient. This cushioning pad may be made of any material, including gel material, or a foam pad. A plurality of cushioning pads may be incorporated in the arm support region 111, to support different regions of the arm 4 of the patient. Alternatively, a number of cushioning pads may enable the arm support 100 to be used on various size arms. The cushioning pads may come pre-attached to the arm support strap 101. Alternatively, they may be placed on the arm support strap 101 before use, for instance using adhesive attachments, allowing unconstrained placement of the arm 4 in any location of the arm support region 111. Alternatively, rather than cushioning pads, the arm support may be made of fabric with different weaves, for indicating the placement position for the arm.

Instead of, or in addition to, cushioning pads, there may be an elbow registration slot on the arm support region 111 for receiving the elbow of the patient. This may provide a reference point for positioning the arm. The elbow support slot may be any size or shape of aperture, including v-shaped, and may include a slot or cut out region. There may also be provided elbow registration slots along the width and/or length of the arm support strap 101, for instance comprising a series of multiple weakness points in the arm support region 115. Advantageously, having multiple elbow registration slots 115 has the advantage that various sizes of arms can be accommodated.

The thumb hole 121 as shown in Figures 1 and 4 has a circular profile. However, any other shape could be envisaged. For instance, the thumb hole 121 may have a circular profile. In the embodiments shown, there is only one hand support strap 115 which either anchors the thumb, or the palm, of the patient. However, more than one hand support strap 115 could be used. For instance, one hand support strap 115 may anchor the thumb, and the second hand support strap 115 may anchor the palm. Advantageously, this provides further securing of the hand in dorsiflexed position. In the embodiment shown in Figures 1 and 3, the hand support strap 115 anchors the thumb using a thumb hole 121, however the thumb may be anchored by wrapping the thumb receiving end 119 around the thumb of the patient. This would achieve the same anchorage to the thumb. Alternatively, rather than just anchoring the thumb, some, or all, of the fingers of the hand may be anchored in the same way as described in relation to the thumb above.

In the embodiments described above, the arm support strap 101, and the secondary support strap 300 (when used), are attached to the board 200 underneath the patient. Alternatively, the straps may be attached directly to the bed on which the patient is lying. In other situations the straps may be attached directly to the patient. This would provide the advantage that if the patient were to sit up, the arm/arm support system would move with the patient ensuring that the arm positioning is kept constant relative to the body.

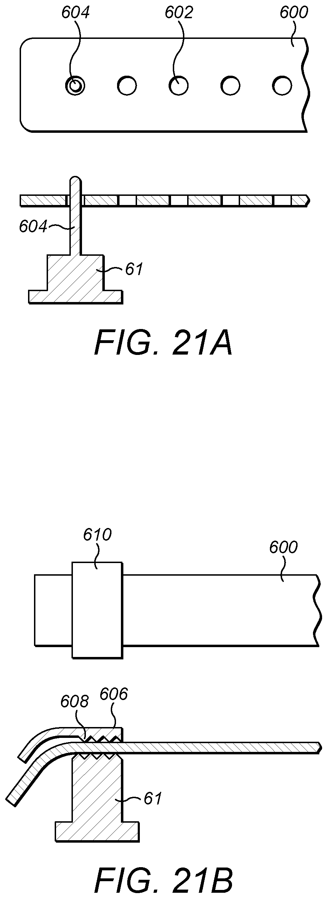

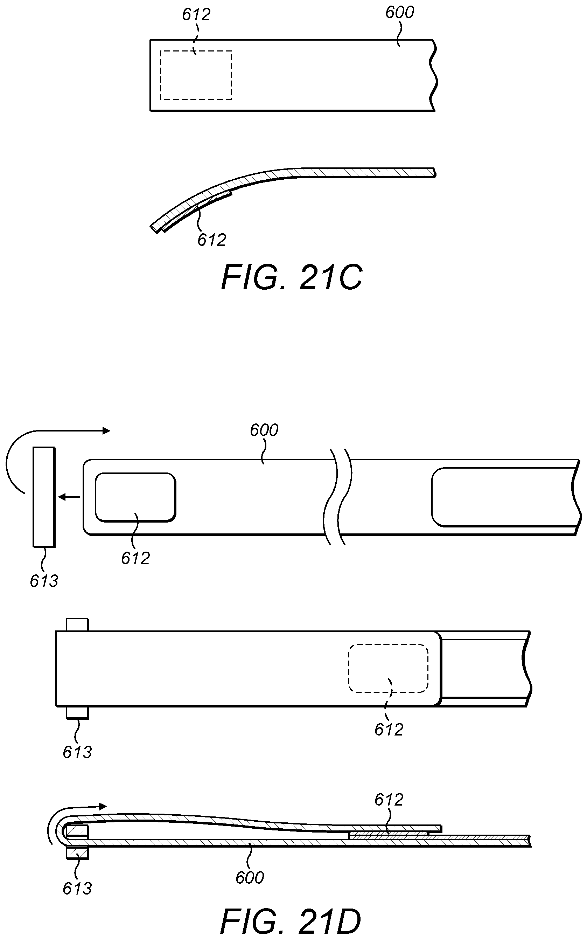

Although the attachments are preferably hook and eye fasteners, any other attachment or combinations of attachments may be used in combination with one another, as will be described in relation to Figure 21 below.

As shown in Figures 1 and 4, the adhesive strips 113, 307 and 303 comprise three elongate strips of adhesive material. However, any number of strips of adhesive material may be used. Advantageously, having strips of adhesive material allows easy detachment and repositioning of the arm support 101. Alternatively, rather than having separate strips, one large patch of adhesive material may be used, as this provides a more secure attachment than separate strips. Any combination of adhesive patches may be used. For instance, in one example arm support 100 there may be three adhesive strips 113, four adhesive strips 307, and one large patch of adhesive material 303. Although only one adhesive strip is shown for 105, 109, 301, 305, 117 a plurality of adhesive strips may be used for securing to the corresponding attachments. Alternatively, it can be understood that any of the plurality of adhesive patches 113, 307, 303 could be one adhesive strip, and any of the adhesive strips 105, 109, 301, 305, 117, may be a plurality of strips.

Although in the embodiment shown above adhesive strips 113, 307, 303 are orientated perpendicular to adhesive strips 105, 109 and 305; they may be orientated at any angle to one another. When in use, not every one of the plurality of attachments 113, 307 and 303 has to be attached to the corresponding strips 105, 109 and 305. A secure attachment, that is more comfortable for the patient, may be provided when only some of the plurality of adhesive strips 113, 307 and 303 are attached to strips 105, 109 and 305.

An alternative packaging and dispensing means to the roll 400 illustrated in Figure 16 could be in the form of a box comprising a series of arm supports 100. The arm supports may be stored within the box with a portion of the arm support 100 projecting from the box. Dispensing of an arm support 100 from the box could be achieved by pulling the protruded portion of the arm support 100 from the box, and in carrying out this action the next arm support 100 is pulled upwards so that the next arm support 100 projects out from the box. This is similar to the method of dispensing tissues that is commonly known.

Whether packaged in the form of a roll 400 or a box, the plurality of arm supports 100 may be provided together as a package with an associated attachment means. By way of example, for an arm support 100 using adhesive to secure it in position, the package may include a suitable number of adhesive strips for application to arm support 100. Alternatively, where the attachment comprises a clamp, a rod or spike, or a slot, and/or a board (as will be described in more detail in relation to Figure 21 below), then the package may include that securing attachment.

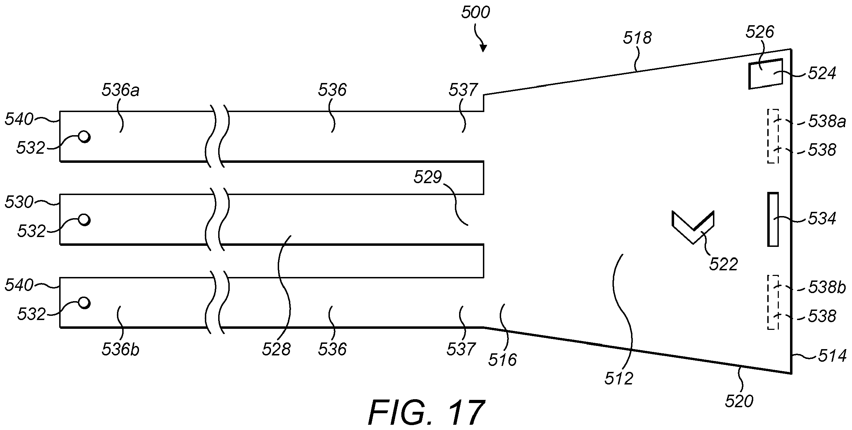

Figure 17 illustrates an arm support 500 according to a second embodiment of the present invention, showing a 'blank' of the arm support 500 in a flat condition when not in use.

The arm support 500 comprises an elbow support region 512 that is for receiving the arm of a patient. The elbow support region 512 comprises a lateral side 514, a medial side 516, a wrist end 518 and a shoulder end 520.

The elbow support region 512 further comprises a v-shaped slot 522 for registering the elbow of the patient in a position on the elbow support region 512, as will be described more fully below. The v-shaped slot 522 is located just off centre of the elbow support region 512, slightly towards the lateral side 514.

The arm support 500 further comprises a thumb-receiving portion 524 for receiving the thumb of the patient. The thumb-receiving portion 524 comprises an opening 526 in the arm elbow support region 512 on the lateral side 514 towards the wrist end 518 of the arm support 500. The opening 526 has a rectangular shape, but alternative shapes are envisaged, such as a circle, oval, or a simple slit.

An upper anchor strap 528 extends from a proximal end 529 at the medial side 516 of the elbow support region 512, approximately in line with the elbow registration slot 514, to a free, distal end 530. The upper anchor strap 528 has an elongate profile, and preferably the length of the upper anchor strap 528 is greater than the length and width of the elbow support region 512 such that the free end 530 of the upper anchor strap 528 is suitable for extending over the body of the patient 2 when in use. An attachment means 532 is provided towards the free end 530 of the upper anchor strap 528 for securing the arm support 500 in position when in use, as will be more fully described below.

The upper anchor strap 528 has an associated upper anchor slot 534 in the elbow support region 512, for receiving the upper anchor strap 528. The upper anchor slot 534 is located on the lateral side 514 of the arm support 500, opposite to the upper anchor strap 528. The upper anchor slot 534 has a width and depth sufficient to allow at least the free end 530 of the associated upper anchor strap 528 to pass through it.

As can be seen from Figure 17, the arm support 500 also has two external rotator straps 536. The external rotator straps 536 extend from respective proximal ends 537 on the medial side 516 of the arm support 500, one either side of the upper anchor strap 528, to respective free, distal ends 540. A fore external rotator strap 536a is located on one side of the upper anchor strap 528, closest to the wrist end 518 of the arm support 500. A rear external rotator strap 536b is located on the other side of the upper anchor strap 528, closest to the shoulder end 520 of the arm support 500. In the embodiment shown in Figure 17 the external rotator straps 536 have substantially the same shape and width as the upper anchor strap 528.

A fore external rotator strap slot 538a and a rear external rotator strap slot 538b are provided either side of the upper anchor slot 534, for receiving the fore and rear external rotator straps 536, respectively. The external rotator slots are located on the lateral side 514 of the arm support 500, opposite their respective external rotator straps 536. The external rotator slots 538 are openings in the elbow support region 512 with a width and depth sufficient to allow at least the free end 540 of the respective associated external rotator strap 536 to pass through it.

The external rotator straps 536 are each provided with attachment means 532 towards the free end 540 for securing the arm support 500 when in use, as will be described in greater detail below.

The arm support 500 comprising the elbow support region 512, thumb-receiving portion 524, upper anchor strap 528, and external rotator straps 536, is formed from one continuous, unitary piece of material. Advantageously, this means that the arm support 500 can be manufactured and provided cheaply, and in bulk, requiring little assembly before use.

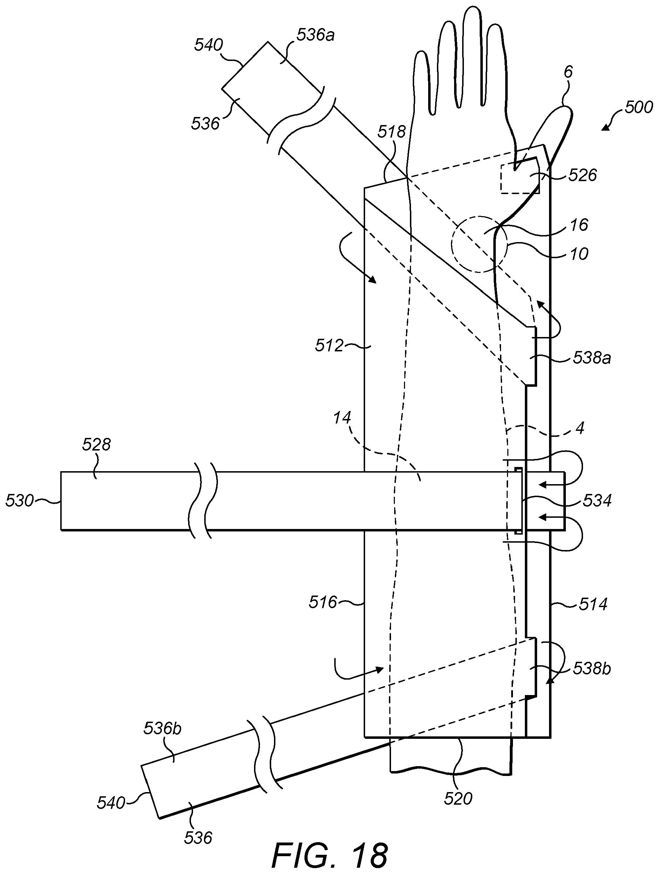

Figure 18 illustrates the arm support 500 according to the embodiment shown in Figure 17 in use supporting a patient's right arm 4. As can be seen from Figure 18, the patient's arm 4 is received on the elbow support region 512; the arm 4 in a straight orientation extends down the length of the arm support 500. The patient's elbow is registered within the v-shaped slot 522 of the elbow support region 512 thereby providing a reference point ensuring the arm 4 is located in the correct position on the arm support 500, whilst helping to reduce movement of the arm 4 by maintaining the arm 4 in the correct position. This makes the arm support 500 intuitive to use, such that medical personnel, if not already familiar with the arm support 500, will be able to easily identify how the arm support 500 should be applied to the arm 4. Advantageously, the v-shaped slot 522 further aids in providing greater flexibility of the arm support 500, increasing its functionality for receiving a wide range of arm sizes. The registration slots or cushioning pads as described for the previous embodiment may also be used.

The patient's thumb 6 is received through the opening 526 of the thumb-receiving portion 524. The thumb-receiving portion 524 provides anchorage of the thumb 6 to prevent it from moving, whilst providing attachment between the arm 100 and the arm support 500. By virtue of the location of the patient's elbow in the elbow registration slot 522 and the thumb 6 within the opening 526, the patient's wrist 16 is urged into a dorsiflexed configuration, such that the radial site 10 is easily accessible.

The securing and orientation of the arm 4 is provided by the series of straps 528, 536. As can be seen from Figures 18 to 20, in use, the upper anchor strap 528 is passed over the patient's arm 4 from the medial side 516 to the lateral side 514 and specifically over the patient's antecubital 14 (the inside bend of the elbow), then the free end 530 thereof is passed through the associated upper anchor slot 534. This brings the medial side 516 of the elbow support region 512 up and over the arm 4 towards the lateral side 514, at least partially encircling the arm in the process. The upper anchor strap 528 is then passed back over itself, over the top of the arm/arm support system and across the torso 8 of the patient. The free end 530 of the upper anchor strap 528 is then secured to a portion of the bed frame 542 on the opposite side of the patient's torso 8 to the arm 4.

The external rotator straps 536, in use, each pass over the arm 4 and through their associated external rotator slot 538. The external rotator straps 536 then pass underneath the arm/arm support system. The free end 540 of each external rotator strap is then secured to the bed frame 542. As can be seen in Figure 18, the fore and rear external rotator straps 536a, 536b when passing under the arm/arm support system extend in a direction away from each other. More specifically, the fore external rotator strap 536a passes diagonally underneath the arm/arm support system towards the wrist end 518 of the elbow support region 512, and the rear external rotator strap 536b passes diagonally underneath the arm/arm support system towards the shoulder end 520 of the elbow support region 512.

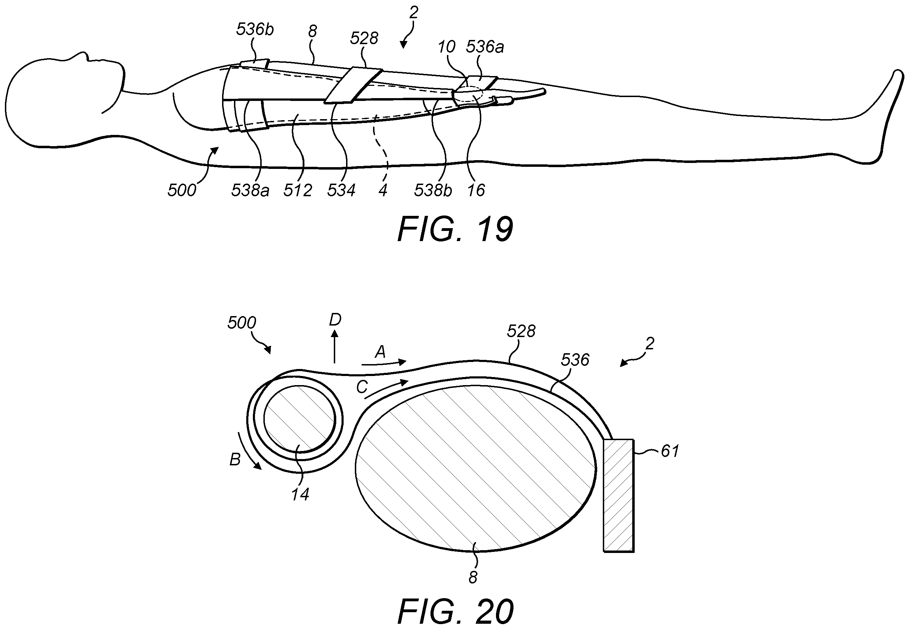

Figure 19 illustrates how the straps of the arm support 500 attach in relation to the body of the patient 2. Figure 19 clearly shows the free end 530 of the upper anchor strap 528 passing above the arm/arm support system and subsequently across the torso 8 of the patient. The external rotator straps 536 are also shown in Figure 19, showing the free ends 540 thereof passing under the arm/arm support system and subsequently over the torso 8 of the patient. The fore external rotator strap 536a passes over the lower end of the torso 8 of the patient, and the rear external rotator strap 536b passes over the upper end of the torso 8 of the patient.

The specific arrangement of the straps of the arm support 500 ensures favourable positioning and support of the arm 4 throughout the radial access procedure. The passing of the upper anchor support across the patient's arm 4 through the associated upper anchor slot 534 and then back on itself causes the arm support 500 to form around the arm 4 of the patient due to the flexible nature of the arm support 500. The passing of the free end 530 of the upper anchor strap 528 across the torso 8 of the patient to be secured under tension causes the arm 4 to be secured in a raised elevated position substantially co-planar with the torso 8, tight against the side of the body of the patient 2. The location of the arm 4 is typically within one arm thickness of the top of the patient's torso 8 when they are lying down as in Figure 19 and 20, secured anywhere between either one arm 4 thickness above the top of the torso 8 or one arm 4 thickness below the top of the torso 108 in this orientation. The relatively high position of the arm 4 in relation to the body of the patient 2, advantageously provides the physician with an improved operating position.

Whilst the upper anchor strap 528 anchors the arm 4 in a preferable location relative to the patient's body 2, the external rotator straps 536 ensure that correct orientation (external rotation) of the patient's arm 4 is achieved. The fore and rear external rotator straps 536 pass underneath the arm/arm support system and pass across the patient's torso 8 providing tension to orientate the arm 4 in a specific way, whilst not obstructing access to the radial site. The fore external rotator strap 536a provides tension to align the wrist 16 in a horizontal, externally rotated position. In conjunction with the thumb-receiving portion 524, the fore external rotator strap 536a causes rotation of the hand to ensure that the arm 4 is in a dorsiflexed position, with the underside of the arm and palm facing upwards. The combination of the fore external rotator strap 536a and the thumb-receiving portion 524 ensures that the radial site 10 is easily accessible during the procedure whilst maintaining the arm 4 in a fixed position. The rear external rotator strap 536b also ensures that the arm 4 is maintained in a straight orientation providing tension to the side of the arm 4 above the elbow, such that there is uninterrupted access along the length of the arm 4 during the procedure.

The fore and rear external rotator straps 536a, 536b may also provide additional support to the arm 4, assisting the anchor strap 528 in securing the arm 4 in the raised position tight against the patient's side and substantially in line between the middle and the top of the torso 8.

A cross section showing a free body diagram of the strap arrangement of the arm support 500 in use, relative to the body of a patient 2, is illustrated in Figure 20. Figure 20 shows the forces provided by the straps due to their specific orientation and securement. Force A is the tension provided by the upper anchor strap 528 extending over the arm support 500 and back over the arm/arm support system and secured across the torso 8 of the patient. Force B is the tension provided by the external rotator straps 536 passing under the arm/arm support system, which causes the arm to rotate externally so as to position the wrist in the correct dorsiflexed orientation described above. The external rotator straps 536 also provide tension, labelled here as Force C, by virtue of their being passed across the patient's body 2 to be secured in position. The tension provided by the forces through the straps can be resolved into horizontal and vertical components. The tension provided by both Force A and C have a horizontal component that urges the arm 4 towards the patient's side, as well as a vertical component that urges the arm 4 upwards, against the weight of the arm which is in the opposing vertical direction. Force D represents the combined effect of the vertical components of the tension provided by the secured straps 528 and 536, resulting in the upwards force that maintains the arm 4 in the preferred high orientation relative to the torso 8 of the patient. Both the upper anchor strap 528 and the external rotator straps 536 after passing across the torso 8 of the patient are attached by securing to the bed frame 61 on the opposite side of the patient's body to the arm that is being supported. The weight of the patient's torso is supported by the bed on which the patient is laying (not shown in Figure 20). The arrangement of the straps 528 and 536 across the torso of the patient helps to distribute the load of the patient's arm to the bed, which is maintained in position by the securing to the bed frame 61.

Various securing devices suitable for attaching the upper anchor strap 528 and the external rotator straps 536 to the bed frame 542 are illustrated in Figure 21.

According to the embodiment shown in Figure 21A, the strap 600 (which may represent any of the anchor strap 528 or the external rotator straps 536) comprises a series of holes 602 at least over a portion towards the free end 530, 540 thereof for co-operation with an anchor post 604 attached to the bed frame 61. The arm support 500 can be attached securely by pushing a selected hole 602 in the strap 600 on to the anchor post 604, with tension maintained by this attachment. Advantageously, having a series of holes 602 the arm support 500 can be used to accommodate patients of a variety of sizes.

An alternative means for securing the strap 600 is shown in Figure 21B, comprising a clamp 606 connection between the strap 600 and the bed frame 61. The strap 600 is fed through a clamp 606 with gripper teeth 608 of the clamp 606 engaging with a gripping region 610 of the strap 600. For instance, the clamp 606 may be opened such that the gripper teeth 608 are clear of the strap 600 for feeding at least the free end 530, 540 thereof through the clamp 606. When the desired tension is achieved in the strap 600, the clamp 606 may be closed back into position, with the gripping teeth 608 gripping the gripping region 610 of the strap 600 to ensure secure attachment and maintain the tension in the strap 600. The gripping region 608 may be reinforced.

An alternative means for securing the strap 600 is shown in Figure 21C, comprising an adhesive attachment. In this embodiment, the strap 600 has an adhesive patch 612 at its free end suitable for sticking to the bed frame 61. Various sizes of patients can be accommodated as the adhesive patch 612 can be adhered to different regions of the bed frame as necessary, depending on how far from the elbow support region 512 the free end 530, 540 of the strap 600 extends. In alternative embodiments a series of adhesive patches 612 may be provided, or one long continuous adhesive patch 612, to ensure that a wide variety of sizes of patients are accommodated. Advantageously, an adhesive patch 612 provides quick and easy attachment of the arm support 500 whilst maintaining secure support.

An alternative means for securing the strap 600 is shown in Figure 21D, comprising a slot 613 on the bed frame 542 and an adhesive patch 512 for attaching the strap onto itself. In this embodiment, the strap 600 has an adhesive patch 612 at its free end suitable for sticking onto itself. The free end of the strap 600 is fed through the slot 613 on the bed 61, and then back over itself. When the desired tension is achieved, the free end of the strap 600 is attached to itself by the adhesive patch 612, holding the strap 500 in place. The slot 613 may be provided in a component that is secured to the bed frame 61, or may be an integral part of the bed frame 61 alternatively it may be provided on a board 200 as shown in Figure 2.

The adhesive patch used in the means for securing shown in Figures 21C and 21D can be a hook and eye fastener, sticky tape, or any other type of adhesive means known in the art.

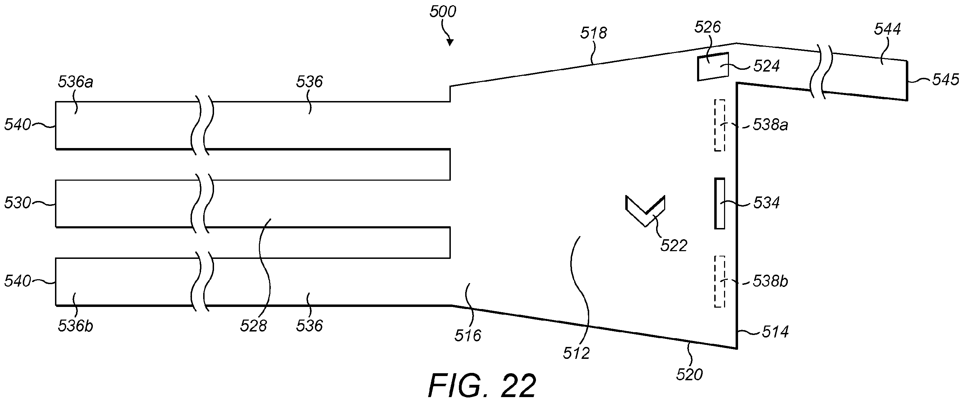

Although, Figure 21 is described here in relation to the second embodiment shown in Figures 17 and 22, the attachments could also be used for attaching the second end 107 of the arm support strap 101 and/or the secondary support strap 300, as shown in the embodiments in Figures 1 to 16.

An alternative, third embodiment of the arm support 500 is illustrated in Figure 22 showing a 'blank' of the arm support 500 in a flat condition when not in use. In this embodiment, the thumb-receiving portion 524 comprises the additional feature of a thumb strap 544. The thumb strap 544 is located adjacent to the opening 526 of the thumb-receiving portion 524 on the lateral side 514 of the arm support 500. After the thumb 6 has been received through the opening 526 of the thumb-receiving portion 524 the thumb strap 544 is wrapped around the thumb. The free end 545 of the thumb strap 544 is then passed under the arm/arm support system and pulled under tension before being secured in position for example by any of the above-described securing means of Figure 21. The securing means ensures that the thumb strap 544 remains under tension. Preferably, the attachment is performed after the free end 545 of the thumb strap 544 has passed across the torso 8 of the patient and is attached to the bed frame 61.

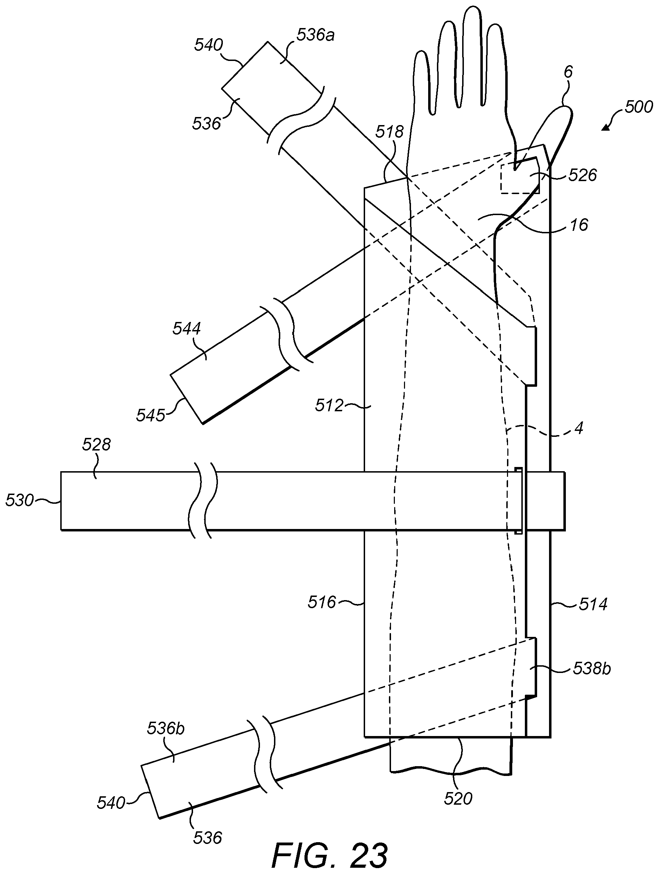

As shown in Figure 23, the free end 545 of the thumb strap 544 passes under the arm/arm support system in a direction substantially perpendicular to the fore external rotator strap 536a, about parallel to the direction of the extended thumb, i.e. diagonally in a direction towards the medial side 516 and shoulder end 520 of the arm support 500. Advantageously, the thumb support strap 544 provides additional wrist support and assists in properly orienting the wrist in the dorsiflexed position, furthermore passing under the arm/arm support system provides clear access to the radial site.

The thumb strap 544 anchors the thumb, causing the thumb 6 to be fixed in a position extending away from the hand due to the direction that the thumb strap 544 passes under the arm support 500. Advantageously, the anchorage provided by the thumb strap 544 ensures that the hand remains in one straight position providing clear access to the radial site 10.

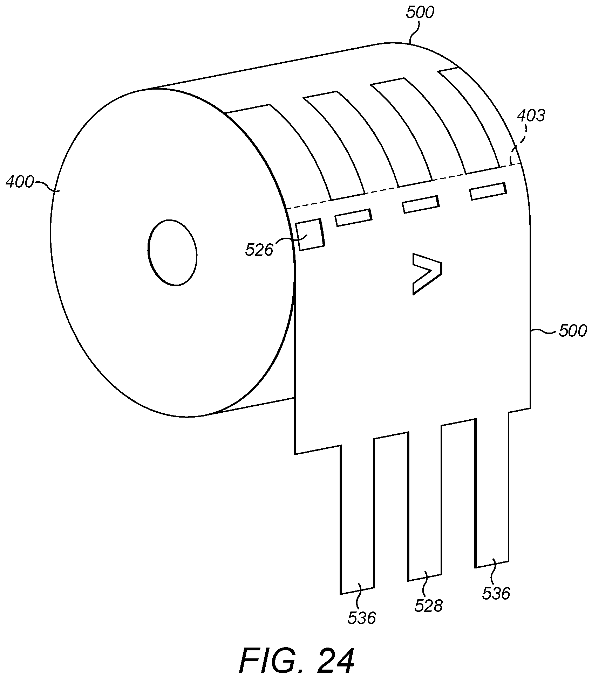

A number of arm supports according to the embodiments described can be provided on a roll 400 as illustrated in Figure 24. The roll 400 may be dispensed using the roll dispenser 401 as shown in Figure 16A. The roll 400 comprises a series of arm support blanks, attached together with perforations 403 or some other line of weakness between adjacent arm supports 500 to provide easy detachment from the roll 400 when required. Upon pulling an arm support 500 from the roll 400, the perforations 403 provide a line of weakness in the material resulting in the arm support 500 being detached from the roll 400. Preferably, the arm supports are formed from the same piece of material. In one embodiment the roll 400 can hold five arm supports. In another embodiment, the roll 400 may hold fifty arm supports. Advantageously, this provides easy transport of the arm supports and convenient dispensing in the surgical environment. The features of the arm support 500, such as the opening 526 of the thumb-receiving portion 524, may already be cut out on the arm support blanks. However, they may alternatively be provided as perforations to be pushed out after dispensing of the arm support 500 from the roll 400.

During the radial access procedure a catheter is inserted into the radial artery in the patient's arm 4. Advantageously, the arm support 500 according to the described embodiments provides a clear distal zone for inserting the catheter into the arm 4, with the thumb receiving-portion 524 and the external rotator straps 536 orientating the arm 4 such that the radial artery is retained easily accessible throughout the procedure.

Preferably the arm support 500 is made from hypoallergenic, low weight, flexible, absorbent material. The material preferably has an absorbent upper surface and a waterproof under surface that resists fluid-strike through the material, and prevents body fluid run off during the procedure. Preferably the arm support 500 is disposable, reducing the risk of infections. Preferably the arm support 500 is made from a non-woven, low linting material that is strong in wet and dry conditions. Preferably it will meet flammability class 1. Preferably it will be a material such as Steri-Drape™.

During the radial access procedure monitoring of the catheter is provided by x-rays or other radiation detection methods, therefore the arm support 500 is preferably made from a radiolucent material to enable full visualisation of the heart through the arm support 500 by fluoroscopy.

Advantageously the arm support 500 is provided with straps so that there is easy access for defibrillator pads and ECG lead electrode attachments to the chest, for monitoring and treatment of electrical disturbance during the interventional procedure. The defibrillator pads may be applied before securing the arm support 500 to the patient. Advantageously, performing defibrillation whilst the arm is located in the arm support 500 prevents the arm from flailing around during shocks which could damage equipment. Moreover, the attachment means may permit quick removal of the arm support 500 before defibrillation, for instance if adhesive patches are used, such as hook and loop fasteners, the straps may also be easily cut.

It is intended that the arm support 500 is reversible for use on both the left and right arms. As can be identified from Figures 17 and 22, most of the functional features are the same on both the front and reverse sides. To ensure reversibility of all embodiments, the means for securing the respective straps needs to be functional in both orientations of the arm support 500. For instance, in the embodiment shown in Figure 21C the adhesive patch 612 can comprise double sided tape that is attached to the straps after selecting the orientation required for the arm support: i.e. for use with a right arm, the adhesive patch 612 would be stuck on the top side of the free end of the strap 600 as illustrated, but for use with a left arm it would be stuck on the underside instead (which would be the top side when reversed for use on that left arm). In another embodiment, there may be provided an adhesive patch 612 on both front and reverse of the arm support 500 blank, with only a select one of those being used, depending on the orientation. Alternatively, the embodiment shown in Figure 18B may have a gripping region 610 on both sides of the straps. The features of the arm support 500 are therefore adaptable such that the reversible nature of the arm support 500 is achieved.

A further function of the arm support 500 is that post surgery it can be readily adapted to be used as a sling adapted to support the arm 100 relative to the patient. For instance, the external rotator straps and upper anchor strap can be detached from the bed frame. The straps are then passed around the neck of the patient and attached to one another, forming a sling.

The arm support 500 according to embodiments provides passive support to the patient even when unconscious, providing support from the underside of the arm support 500 with the load from the arm 4 distributed by the straps when under tension. Advantageously, rather than being attached directly to the bed frame 61 on the same side of the patient's body 2 as the arm 4 that the procedure is being carried out on (as is common in current radial access techniques), the arm support 500 provides attachment over the body 2 of the patient ensuring the arm 4 is always maintained in the same plane as the patient's body 2. This additionally causes the arm 4 to be raised and supported in a high position against the patient's body 2, rather than located in a low orientation attached directly to the bed 60. This provides the advantage of the arm 4 being at a more desirable operating position for the physician, lowering their risk of developing back complaints from carrying out multiple procedures over time. Furthermore, due to the high arm 4 position and the straps, surgical drapes across the arm and torso are approximately flat, or may even have a dip in them, in sharp contrast to the existing situation in which drapes have a tendency to form a tent-like surface from which any surgical tools or equipment that may be rested on the drapes are prone to sliding off.

Advantageously, passing the straps of the arm support 500 over the body of the patient 2, before being attached to the bed frame 61, helps to prevent the patient from sitting up during the procedure.

Advantageously, the arm support 500 can be used on patients of any size with the arm support 500 adaptable to specific patients, unlike the systems used in current radial access techniques. This is achieved by the external rotator straps 536 and upper anchor strap 528 having a length sufficient to secure any patient size with attachment means ensuring the desired tension can always be achieved.

Many modifications and variations may be made to the above-described embodiments within the scope of the invention.

The elbow support region 512 illustrated in Figures 17 and 22 has a substantially trapezoidal shape. However, the elbow support region 512 may be of any shape that provides a supporting cradle around the arm 4. Furthermore, the wrist end 518 of the arm support 500 may have a cutaway portion. Advantageously, this provides access to the hand and wrist of the patient allowing easy cleaning and draping.

The elbow registration slot is not necessarily required to be a v-shaped opening, and any alternative size or shape aperture, slot or cut-out of the elbow support region 512 may be used. There may also be provided elbow registration slots along the length of the arm support 500, from the wrist 518 to the shoulder end 520, for instance comprising a series of multiple weakness points in the elbow support region 512. Advantageously, having multiple elbow registration slots 522 has the advantage that various sizes of arms can be accommodated; the distance between the thumb-receiving portion 524 and the elbow registration slot 522 being important to define a proper fit to the patient.

The opening 526 on the thumb-receiving portion 524 does not have to have a rectangular profile and any other shape could be envisaged. For instance, the opening 526 of the thumb-receiving portion 524 may have a circular profile. In the embodiment disclosed in Figures 22 and 23 the thumb-receiving portion 524 comprises both an opening 526 and a thumb strap 544. However, in an alternative embodiment the thumb-receiving portion 524 may comprise only a thumb strap 544 with no opening 526. In a further embodiment the thumb strap 544 does not necessarily have to wrap around the thumb, with the thumb strap 544 just providing anchorage to the thumb. The thumb strap 544 may, rather than being formed on the lateral side 514 of the arm support 500 from the thumb-receiving portion 524, branch off from the fore external rotator strap 536a, such that the one external rotator strap provides the function of the fore external strap in addition to the function of a thumb strap 544. In alternative variants of this embodiment, rather than having a thumb receiving portion 524, a palm receiving portion, for instance a palm strap may be provided to anchor the arm into the dorsiflexed orientation.

In the embodiments described above the upper anchor strap 528 and external rotator straps 536 are attached to the bed after passing across the torso 8 of the patient. However, the straps may be attached directly to the patient, for instance by adhesive patches. This would provide the advantage that if the patient were to sit up, the arm/arm support system would move with the patient ensuring that the arm 4 positioning is kept constant relative to the body 2. Alternatively, the upper anchor strap 528 and external rotator straps 536 may, after passing across the torso 8, be positioned underneath the torso 8, between the torso 8 and the bed that the patient is on, with the weight of the patient's torso 8 maintaining the tension in the straps of the arm support 500 to hold the arm in place.

Alternatively, various orientations of the straps may be implemented to ensure the same effect. For instance, the external rotator straps 536, rather than passing over the bed, may pass under the bed before being attached, or in some embodiments they may attach to the same side of the bed that the arm/arm support system is located on. Advantageously, having straps attaching both sides of the patient's body can prevent the dislodging of surgical instruments from the unwanted movement of the patient during the procedure.

Although two external rotator straps 536 are provided in the embodiments discussed above, any number of external rotator straps 536 could be used. For instance in one embodiment only one external rotator strap 536 may be provided, or in a further embodiment more than two external rotator straps 536 may be provided. Furthermore, more than one upper anchor strap 528 could also be used to provide an improved secured attachment. The location of the upper anchor strap 528 relative to the external rotator straps 536 may be provided with various arrangements. For instance, two external rotator straps 536 may be located next to one another with no upper anchor strap 528 located in between. This may occur when there are more than two external rotator straps 536. The external rotator strap slots 538a, 538b are optional. The free ends 540 of the external rotator straps 536 may instead be passed over the top of the lateral side 514 before passing under the elbow support region 512. However, the provision of the external rotator strap slots 538a, 538b helps to position and orient the external rotator straps 536.

Rather than being located in the position on the arm support 500 as shown in the figures, each external rotator strap 536 in an alternative embodiment may be located on the lateral side 514 of the arm support 500, with no external rotator slot 538 being provided. In this embodiment, the free end 540 of the external rotator strap 536 passes directly underneath the arm/arm support system and is secured by any of the previously described means.

The size of the straps may be tailored to suit the method and location of attachment. For instance, in embodiments where the upper anchor strap 528 is attached after passing across the patient and the external rotator strap 536 is attached on the side of the bed that the arm/arm support system is located on then the external rotator straps 536 may be significantly shorter than the upper anchor strap 528, as the external rotator straps 536 do not have to accommodate the size of the patient and have less distance to extend before being secured. However in other embodiments where both the upper anchor strap 528 and the external rotator straps 536 pass across the torso 8 of the patient they may have substantially similar lengths.

No matter what combination of straps and associated slots, or lack thereof, the arm support 500 must perform the function of holding the patient's arm close to the side of the patient's body 2, raised against the patient's torso 8, whilst presenting the wrist 16 in a dorsiflexed configuration.

The securing devices illustrated in Figure 21 can be used to provide the attachment for the upper anchor strap 528 and external rotator straps 536. Any combination of attachment means may be used in combination with one another. For instance, in one embodiment the upper anchor strap 528 may have holes 602 for being received by an anchor rod 604 on the bed according to Figure 21A, and the external rotator straps 536 may have adhesive patches 612 according to Figure 21C for attachment to the bed frame or to the patient's body. Alternatively, the straps may comprise multiple attachment means. For instance, the upper anchor strap 528 may have both holes 602 and an adhesive patch 612 for providing attachment. Having numerous attachment means advantageously provides a more secure attachment or more options to the surgical team for proper orientation and positioning (and securement locations) for the various straps.

In a further alternative embodiment, the upper anchor strap 528 may not comprise any specific means of attaching and the securing device may be provided solely by an anchor rod or spike located on the bed. In this embodiment, attachment is achieved by pressing the upper anchor strap 528 over the rod to pierce a hole into the strap 528. This alternative attachment means may also be used for the external rotator straps 536 and for the thumb strap 544, where provided. Advantageously, not having pre-formed holes 602 in the strap 600, and being able to puncture holes 602 into the strap 600 where required, enables the strap 600 position to be tailored when in use to meet the exact requirement for the patient's profile.

An alternative packaging and dispensing means to the roll 400 illustrated in Figure 24 could be in the form of a box comprising a series of arm supports 500. The arm supports may be stored within the box with a portion of the arm support 500 projecting from the box. Dispensing of an arm support 500 from the box could be achieved by pulling the protruded portion of the arm support 500 from the box, and in carrying out this action the next arm support 500 is pulled upwards so that the next arm support 500 projects out from the box. This is a similar to the method of dispensing tissues that is commonly known.

Whether packaged in the form of a roll 400 or a box, the plurality of arm supports may be provided together as a package with an associated attachment means. By way of example, for an arm support using adhesive to secure the straps in position, the package may include a suitable number of adhesive patches for application to the straps. Alternatively, where the securing means comprises a clamp, a rod or spike, or a slot, then the package may include that securing means for attachment to the bed frame.

Other embodiments of the invention will be apparent to those skilled in the art from consideration of the specification and practice of the embodiments disclosed herein. It is intended that the specification and examples be considered as exemplary only, with the scope of the invention being indicated by the following claims.

## Claims

1. An arm support (100) for use in a medical procedure, the arm support comprising:
an arm support strap (101) comprising:
a first end (103) that is configured to pass around the arm of a patient and secure to a region of the arm support strap;
a support region (111) for supporting an arm of the patient; and
a second end (107) configured to pass across the torso of the patient to be secured so as to support the arm securely against a side of the torso in a position substantially co-planar with the torso; and **characterised in that** the arm support further comprises:
a hand support (115), comprising:
a first region (117) adapted to secure the hand support to the arm support strap; and
a second region adapted to engage with a hand of the patient for securing an associated forearm of the patient in a supinated, externally rotated position.

2. The arm support (100) according to claim 1, wherein the second region of the hand support (115) comprises a thumb receiving portion (119), which preferably comprises an opening (121) in the second region of the hand support.

3. The arm support (100) according to claim 2, wherein the thumb-receiving portion (119) comprises a thumb strap configured to pass underneath the hand and secure the thumb in position.

4. The arm support (100) according to any of claims 1 to 3, wherein the first region (117) of the hand support (115) is removably attachable to the arm support strap (101).

5. The arm support according to any of claims 1 to 3, wherein the hand support (115) and arm support strap (101) are made from a unitary piece of material and are configured to be separable from one another along a line of weakness (123).

6. The arm support (100) according to any of the preceding claims, wherein the arm support (100) is reversible for use on both the left and the right arms.

7. The arm support (100) according to any of the preceding claims, wherein the second end (107) of the arm support strap is configured to be secured to the patient's body, or to a bed or a board (200) under the patient, after passing across the torso.

8. The arm support according to claim 7, wherein the second end (107) of the arm support strap is configured to attach back on itself.

9. The arm support (100) according to any of the preceding claims, wherein the second end (107) of the arm support strap includes an adhesive patch (109) for securing to the patient, or to a bed or board (200) under the patient.

10. The arm support (100) according to any of the preceding claims, further comprising a secondary retention strap (300), the secondary retention strap (300) comprising:
a first end that is configured to be secured to a bed or board under the patient; and
a second end that is configured to attach to the arm support,
such that the secondary retention strap prevents the arm from moving.

11. The arm support (100) according to any of the preceding claims, wherein the second end (107) of the arm support strap can be configured to be attached around the neck of the patient to form a sling; and
wherein the arm support strap (101) comprises a perforation (129) for receiving the head of the patient when forming a sling.

12. The arm support according to any preceding claim, wherein the first end of the arm support strap is configured to pass underneath and then back over the arm of the patient before being secured, and
preferably wherein the region of the arm support strap to which the first end is configured to secure, is a region of the arm support strap that passes across the torso of the patient, and
Preferably wherein the first end (103) of the arm support strap comprises at least one adhesive patch (105) for securing to the region of the arm support strap; and/or
wherein the at least one adhesive patch (105) for securing to the region of the arm support strap is an elongate strip.

13. The arm support (100) according to claim 12, wherein the first end (103) of the arm support strap (101) and said region of the arm support strap each comprise at least one elongate adhesive patch (113) for attaching to one another, wherein the at least one elongate adhesive patch of the arm support strap and said region of the arm support strap are orientated at an angle to one another, whereby the arm support is adjustable to accommodate a range of patient sizes.

14. A package comprising a plurality of arm supports (100) according to any of the preceding claims, preferably wherein the plurality of arm supports (100) is provided on a roll (400), individual arm supports configured to be separable from one another along a line of weakness (403).

15. A kit of parts for use in a medical procedure, comprising:
a package according to claim 14; and
a securing device,
wherein the securing device is configured for securing the second end (107) of the arm support strap (101), and preferably wherein the securing device is for secure attachment to a bed under the patient or to a board under the patient.

## Patentansprüche

1. Eine Armstütze (100) zur Verwendung in einem medizinischen Verfahren, wobei die Armstütze Folgendes beinhaltet:
einen Armstützengurt (101), der Folgendes beinhaltet:
ein erstes Ende (103), das dazu ausgelegt ist, um den Arm eines Patienten herumgeführt und an einem Bereich des Armstützengurts befestigt zu werden;
einen Stützbereich (111) zum Stützen eines Arms des Patienten; und
ein zweites Ende (107), das dazu ausgelegt ist, über den Torso des Patienten geführt zu werden, um so befestigt zu werden, dass es den Arm sicher gegen eine Seite des Torsos in einer Position stützt, die im Wesentlichen komplanar mit dem Torso ist; und **dadurch gekennzeichnet, dass** die Armstütze ferner Folgendes beinhaltet:
eine Handstütze (115), die Folgendes beinhaltet:
einen ersten Bereich (117), der dazu angepasst ist, die Handstütze an dem Armstützengurt zu befestigen; und
einen zweiten Bereich, der dazu angepasst ist, Eingriff mit einer Hand des Patienten zu nehmen, um einen dazugehörigen Unterarm des Patienten in einer supinierten, extern gedrehten Position zu befestigen.

2. Armstütze (100) gemäß Anspruch 1, wobei der zweite Bereich der Handstütze (115) einen Daumenaufnahmeteil (119) beinhaltet, der vorzugsweise eine Öffnung (121) in dem zweiten Bereich der Handstütze beinhaltet.

3. Armstütze (100) gemäß Anspruch 2, wobei der Daumenaufnahmeteil (119) einen Daumengurt beinhaltet, der dazu ausgelegt ist, unter der Hand durchgeführt zu werden und den Daumen in der Position zu befestigen.

4. Armstütze (100) gemäß einem der Ansprüche 1 bis 3, wobei der erste Bereich (117) der Handstütze (115) entfernbar an dem Armstützengurt (101) angebracht werden kann.

5. Armstütze gemäß einem der Ansprüche 1 bis 3, wobei die Handstütze (115) und der Armstützengurt (101) aus einem einzigen Stück Material gefertigt sind und dazu ausgelegt sind, entlang einer Sollbruchlinie (123) voneinander trennbar zu sein.

6. Armstütze (100) gemäß einem der vorhergehenden Ansprüche, wobei die Armstütze (100) zur Verwendung sowohl mit dem rechten als auch linken Arm umkehrbar ist.

7. Armstütze (100) gemäß einem der vorhergehenden Ansprüche, wobei das zweite Ende (107) des Armstützengurts dazu ausgelegt ist, an dem Körper des Patienten oder an einem Bett oder einer Platte (200) unter dem Patienten befestigt zu werden, nachdem es über den Torso geführt worden ist.

8. Armstütze gemäß Anspruch 7, wobei das zweite Ende (107) des Armstützengurts dazu ausgelegt ist, wieder an sich selbst angebracht zu werden.

9. Armstütze (100) gemäß einem der vorhergehenden Ansprüche, wobei das zweite Ende (107) des Armstützengurts ein Klebepflaster (109) zum Befestigen an dem Patienten oder an einem Bett oder einer Platte (200) unter dem Patienten umfasst.

10. Armstütze (100) gemäß einem der vorhergehenden Ansprüche, die ferner einen sekundären Haltegurt (300) beinhaltet, wobei der sekundäre Haltegurt (300) Folgendes beinhaltet:
ein erstes Ende, das dazu ausgelegt ist, an einem Bett oder einer Platte unter dem Patienten befestigt zu werden; und
ein zweites Ende, das dazu ausgelegt ist, so an der Armstütze angebracht zu werden, dass der sekundäre Haltegurt verhindert, dass der Arm sich bewegt.

11. Armstütze (100) gemäß einem der vorhergehenden Ansprüche, wobei das zweite Ende (107) des Armstützengurts dazu ausgelegt sein kann, um den Hals des Patienten angebracht zu werden, um eine Schlinge zu bilden; und
wobei der Armstützengurt (101) eine Perforation (129) zum Aufnehmen des Kopfs des Patienten beim Bilden einer Schlinge beinhaltet.

12. Armstütze gemäß einem vorhergehenden Anspruch, wobei das erste Ende des Armstützengurts dazu ausgelegt ist, unterhalb und dann zurück über den Arm des Patienten geführt zu werden, bevor es befestigt wird, und
wobei der Bereich des Armstützengurts, an dem das erste Ende dazu ausgelegt ist, befestigt zu werden, vorzugsweise ein Bereich des Armstützengurts ist, der über den Torso des Patienten geführt wird, und
wobei das erste Ende (103) des Armstützengurts vorzugsweise mindestens ein Klebepflaster (105) zum Befestigen an dem Bereich des Armstützengurts beinhaltet; und/oder
wobei das mindestens eine Klebepflaster (105) zum Befestigen an dem Bereich des Armstützengurts ein länglicher Streifen ist.

13. Armstütze (100) gemäß Anspruch 12, wobei das erste Ende (103) des Armstützengurts (101) und der Bereich des Armstützengurts jeweils mindestens ein längliches Klebepflaster (113) zum Anbringen aneinander beinhalten, wobei das mindestens eine längliche Klebepflaster des Armstützengurts und der Bereich des Armstützengurts in einem Winkel zueinander ausgerichtet sind, wodurch die Armstütze angepasst werden kann, um für eine Reihe verschiedener Patientengrößen passend zu sein.

14. Eine Packung, die eine Vielzahl von Armstützen (100) gemäß einem der vorhergehenden Ansprüche beinhaltet, wobei die Vielzahl von Armstützen (100) vorzugsweise auf einer Rolle (400) bereitgestellt ist, wobei die einzelnen Armstützen dazu ausgelegt sind, entlang einer Sollbruchlinie (403) voneinander trennbar zu sein.

15. Ein Kit von Einzelteilen zur Verwendung in einem medizinischen Verfahren, das Folgendes beinhaltet:
eine Packung gemäß Anspruch 14; und
eine Befestigungsvorrichtung,
wobei die Befestigungsvorrichtung dazu ausgelegt ist, das zweite Ende (107) des Armstützengurts (101) zu befestigen, und wobei die Befestigungsvorrichtung vorzugsweise zur sicheren Anbringung an einem Bett unter dem Patienten oder an einer Platte unter dem Patienten dient.

## Revendications

1. Support brachial (100) pour une utilisation dans une procédure médicale, le support brachial comprenant :
une sangle du support brachial (101) comprenant :
une première extrémité (103) qui est conçue pour passer autour du bras d'un patient et être fixée à une région de la sangle du support brachial ;
une région de support (111) pour soutenir un bras du patient ; et
une deuxième extrémité (107) conçue pour passer en travers du torse du patient pour être fixée de manière à soutenir fermement le bras contre un côté du torse dans une position essentiellement coplanaire au torse ; et
**caractérisé en ce que** le support brachial comprend en outre :
un support de la main (115) comprenant :
une première région (117) configurée pour fixer le support de la main à la sangle du support brachial ; et
une deuxième région configurée pour mettre en prise une main du patient afin de maintenir un avant-bras correspondant du patient dans une position en supination et en rotation externe.

2. Support brachial (100) selon la revendication 1, où la deuxième région du support de la main (115) comprend une partie recevant le pouce (119) qui comprend de préférence une ouverture (121) dans la deuxième région du support de la main.

3. Support brachial (100) selon la revendication 2, où la partie recevant le pouce (119) comprend une sangle pour pouce conçue pour passer sous la main et maintenir le pouce en position.

4. Support brachial (100) selon l'une quelconque des revendications 1 à 3, où la première région (117) du support de la main (115) peut être attachée de manière amovible à la sangle du support brachial (101).

5. Support brachial selon l'une quelconque des revendications 1 à 3, où le support de la main (115) et la sangle du support brachial (101) sont constitués d'une section unitaire d'un matériau et sont conçus pour être séparables l'un de l'autre le long d'une ligne de plus faible résistance (123).

6. Support brachial (100) selon l'une quelconque des revendications précédentes, où le support brachial (100) est réversible et peut être utilisé sur le bras gauche ou droit.

7. Support brachial (100) selon l'une quelconque des revendications précédentes, où la deuxième extrémité (107) de la sangle du support brachial est conçue pour être arrimée au corps du patient, ou à un lit ou à un panneau (200) sous le patient, après passage en travers du torse.

8. Support brachial selon la revendication 7, où la deuxième extrémité (107) de la sangle du support brachial est conçue pour être attachée en retour sur elle-même.

9. Support brachial (100) selon l'une quelconque des revendications précédentes, où la deuxième extrémité (107) de la sangle du support brachial inclut une pièce rapportée adhésive (109) permettant un arrimage au patient, ou à un lit ou à un panneau (200) sous le patient.

10. Support brachial (100) selon l'une quelconque des revendications précédentes comprenant en outre une sangle de rétention secondaire (300), la sangle de rétention secondaire (300) comprenant :
une première extrémité qui est conçue pour être arrimée à un lit ou un panneau sous le patient ; et
une deuxième extrémité qui est conçue pour être attachée au support brachial, de sorte que la sangle de rétention secondaire empêche tout mouvement du bras.

11. Support brachial (100) selon l'une quelconque des revendications précédentes, où la deuxième extrémité (107) de la sangle du support brachial peut être conçue pour être attachée autour du cou du patient pour former une écharpe ; et
où la sangle du support brachial (101) comprend une perforation (129) pour recevoir la tête du patient lors de la formation d'une écharpe.

12. Support brachial selon l'une quelconque des revendications précédentes, où la première extrémité de la sangle du support brachial est conçue pour passer en dessous puis en retour au-dessus du bras du patient avant d'être fixée et
de préférence où la région de la sangle du support brachial à laquelle la première extrémité est conçue pour être fixée est une région de la sangle du support brachial qui passe en travers du torse du patient et
de préférence où la première extrémité (103) de la sangle du support brachial comprend au moins une pièce rapportée adhésive (105) permettant une fixation à la région de la sangle du support brachial ; et/ou
où la une au moins pièce rapportée adhésive (105) permettant une fixation à la région de la sangle du support brachial est une bande allongée.

13. Support brachial (100) selon la revendication 12, où la première extrémité (103) de la sangle du support brachial (101) et ladite région de la sangle du support brachial comprennent chacune au moins une pièce rapportée adhésive allongée (113) permettant de les attacher l'une à l'autre, où la au moins une pièce rapportée adhésive allongée de la sangle du support brachial et ladite région de la sangle du support brachial sont orientées à un angle l'une par rapport à l'autre par lequel le support brachial est ajustable pour s'adapter à une plage de tailles de patients.

14. Conditionnement comprenant une pluralité de supports brachiaux (100) selon l'une quelconque des revendications précédentes, de préférence où la pluralité de supports brachiaux (100) est fournie sur un rouleau (400), les supports brachiaux individuels étant conçus pour être séparables les uns des autres le long d'une ligne de plus faible résistance (403).

15. Kit de pièces pour une utilisation dans une procédure médicale, comprenant :
un conditionnement selon la revendication 14 ; et
un dispositif de fixation,
où le dispositif de fixation est conçu pour fixer la deuxième extrémité (107) de la sangle du support brachial (101), et de préférence où le dispositif de fixation permet un arrimage sûr à un lit sous le patient ou à un panneau sous le patient.
